# EUROPEAN PATENT APPLICATION

(11) **EP 1 975 171 A1**
(43) Date of publication of application: **01.10.2008**
(21) Application number: 06835187.3
(22) Date of filing: 25.12.2006
(51) Int. Cl.: C07H 15/10, A61K 36/18, A61K 38/00, A61P 35/00, A61P 35/02, A61P 37/04

(54) **ANTI-TUMOR AGENT AND IMMUNOSTIMULATING AGENT**

(30) Priority: 27.12.2005 JP 2005376609
(71) Applicant: University of the Ryukyu, Nakagami-gun, Okinawa 903-0213 (JP); DNA BANK Co., Ltd., Uruma-shi Okinawa, 9042234 (JP); Orion Breweries, Ltd., Urasoe-shi Okinawa 901-2551 (JP)
(72) Inventor: OKU, Hirosuke, Okinawa 9012213 (JP); IWASAKI, Hironori, Okinawa 903-0104 (JP); TAKARA, Kensaku, Okinawa 903-0125 (JP); WATANABE, Hisami, Okinawa 901-2102 (JP); MATSUZAKI, Goro, Okinawa 903-0814 (JP); ISHIKAWA, Takahiro, Okinawa 900-0003 (JP); TAKEUCHI, Kouji, Okinawa 904-2171 (JP); TAIRA, Akira, Okinawa 904-0304 (JP)
(74) Representative: Betten & Resch
(86) International application number: PCT/JP2006/325817
(87) International publication number: WO 2007/074788

(57) **Abstract**

To provide a pharmaceutical composition comprising a ceramide-related substance having little adverse side effects as an active ingredient by using a raw material that is highly safe to a human body and is inexpensive. A pharmaceutical composition comprising at least one sphingoglycolipid derivative that is produced from a beer waste and is represented by the formula below as an active ingredient: wherein R3 represents H or OH; R4 is as defined by (a) or (b): (a) when R3 is H, R4 represents (CH2)13CH3 or (CH2)6CH=CHCH2CH=CH(CH2)4CH3; and (b) when R3 is OH, R4 represents (CH2)yCH3 (where Y represents an integer of 13 to 21) or (CH2)z1CH=CH(CH2)Z2CH3 (where Z1 and Z2 independently represent 0 or a natural number, provided that Z1+Z2=19).

## Description

### [Background of the Invention]

### [Field of the Invention]

The present invention relates to compositions for medicinal purposes that include glycosphingolipid derivatives as active ingredients and have fewer side-effects.

### [Description of the Related Art]

Cancer is a lifestyle-related disease that is a leading cause of death in Japan. Approximately one out of three people who lost their lives as of 2005 died as a result of cancer. At present, establishment of cancer treatment methods and the overcoming of cancer have become high priority issues as health countermeasures in Japan.

In relation to chemotherapy as a cancer treatment method, there exist methods for administering antitumor agents (anticancer drugs and antitumor drugs) inside the body and for destroying tumor cells. At present, a large number of antitumor agents have been developed and used as pharmaceutical agents in practice. Thereby, certain effects have been observed.

As active ingredients of antitumor agents, focus has been placed on ceramide (N-acylsphingosine) and ceramide-related substances in recent years. Ceramide is a type of lipid that exists within a living organism, and it is a substance with a configuration based on an acid amide combination of sphingosine and fatty acids. Additionally, ceramide-related substances are sphingolipids, which have a basic skeleton of ceramide. An example of the aforementioned substance is sphingophospholipid, which is based on a combination of ceramide and sugar, and another example is glycosphingol, which is based on a combination of phosphate and bases. According to the recent studies, it has become apparent that ceramide and ceramide-related substances can function as second messengers that cause tumor cells to induce apoptosis (cell death) via a form of adoption of ceramide and ceramide-related substances within a living organism.

With this as a background, identification of various ceramide-related substances has been undertaken to date. Many technologies using ceramide-related substances as compositions for medicinal purposes have been reported. For instance. Patent Document 1 relates to an invention concerning a method for producing of antitumor agents or immunostimulating agents that contain new glycosphingolipids obtained through ocean surface animals and the like, and concerning the use thereof. Moreover, Patent Document 2 presents an invention relating to compounds having an α-glucosylceramide structure that enhances the immunogenicity of tumor cells and pathogen-infected cells.

As such, the effectiveness of ceramide-related substances as antitumor agents or immunostimulating agents is anticipated. On the other hand, the fact that most of well-known ceramide-related substances show toxicity (cytotoxic activation) even in regards to normal cells (Non-Patent Document 1) has been problematic as a side-effect issue. Such side-effect also constitutes an unavoidable issue for other many antitumor agents used at present. Thus, antitumor agents that surely inhibit the proliferation of tumor cells as targets while having the fewest possible side-effects for normal cells have been desired.

Furthermore, safety relating to raw materials has been problematic with the use of ceramide and ceramide-related substances as antitumor agents as a separate issue. Ceramide-related substances broadly exist in tissues of animals and plants. In particular, many such substances are found relatively frequently in nerve tissues of brains and spinal cords of animals. Thus, conventional ceramide-related substances have been extracted from brains of livestock, such as cows and the like. However, BSE (bovine spongiform encephalopathy) infection has become an international issue. Accompanying such fact, safety in regards to ceramide-related substances obtained through cows' brains has become problematic. Moreover, from the viewpoint of animal protection, new natural raw materials or substitutes for ceramide-related substances that are safe for human bodies in lieu of cows' brains have been desired.

As one of solutions to the raw material issue mentioned above, there exist chemical syntheses of ceramide-related substances. The molecular structures of many of ceramide-related substances have been revealed. Based thereupon, methods of syntheses have also been developed. Therefore, in recent years, use of artificially chemosynthetic ceramide-related substances as substitutes for natural products has commenced. To be sure, artificially chemosynthetic ceramide-related substances can resolve problems related to BSE and animal protection. However, in regards to chemosynthetic ceramide-related substances, harmful drugs remain in bodies treated via chemical syntheses. Alternatively, mixtures of hazardous by-products resulting from the process for synthesis can take place. In light of occurrence of such new problems, it has been difficult to say that chemosynthetic ceramide-related substances are sufficiently safe for human bodies.

At present, plants have been focused upon as raw materials of ceramide-related substances that are highly safe for human bodies. As inventions using plant-derived ceramide-related substances, items using such substances as active ingredients for moisturizers and therapeutic agents for dermatitis and the like are already known. For instance, Patent Document 3 presents an invention relating to therapeutic agents for atopic dermatitis using plant-derived glycosphingolipids as an active ingredient, which are extracted from plants, like konjac starch, by organic solvents. In the case of ceramide-related substances obtained from the aforementioned dietary plants as raw materials, issues relating to the mixture of hazardous by-products resulting from the process for synthesis, as well as issues relating to BSE and animal protection, can be resolved. However, generally speaking, plant cells contain large quantities of glycoglycerolipids. There are small quantities of ceramide-related substances, including glycosphingolipids, in comparison with a case of animals. Thus, a new problem has arisen whereby large quantities of raw materials are necessary in order to obtain a sufficient quantity of ceramide-related substances, and production costs become expensive.
Patent Document 1: Patent No. 3068910
Patent Document 2: International Publication Number WO99/15627
Patent Document 3: Kokai (Jpn. Unexamined Patent Publication) No. 2003-231640
Patent Document 4: Japanese Patent Application No. 2005-272639
Patent Document 5: Kokai (Jpn. Unexamined Patent Publication) No. 11-193238
Patent Document 6: Kokai (Jpn. Unexamined Patent Publication) No. 2004-135053
Non-Patent Document 1: Osman T, Kawamura T, Naito T, Takeda K, Kaer, LV, Okumura K, Abo T (2000) Eur J Immunol, 30, 1919-1928.
Non-Patent Document 2: Fujii H, Seki S, Kobayashi S, Kitada T, Kawakita N, Adachi K, et al. (2005) Virchows Arch, 446, 663-673.
Non-Patent Document 3: Kobayashi E, Motoki K, Uchida T, Fukushima H, Koezuka Y (1995) Oncol Res, 7, 529-534.
Non-Patent Document 4: Kronenberg M (2005) Annu Rev Immunol, 26, 877-900.
Non-Patent Document 5: Taniguchi M, Harada M, Kojo S, Nakayama T, Wakao H (2003) Annu Rev Immunol, 21, 483-513.
Non-Patent Document 6: Osman Y, Kawamura T, Naito T, Takeda K, Kaer LV, Okumura K, Abo T (2001) Eur J Immunol, 31, 1720-1727.
Non-Patent Document 7: Watanabe H, Miyaji C, Seki S, Abo T (1996) J Exp Med, 184, 687-693.

### [Summary of the Invention]

### [Problems to Be Solved by the Invention]

The problem to be solved by the present invention is to develop and supply at a low cost medical compositions as antitumor agents or immunostimulating agents that contain ceramide-related substances as active ingredients with fewer by-products through the use of raw materials that are highly safe for human bodies.

### [Means to Solve the problems]

In order to solve the problem, the present inventors decided to use ceramide-related substances derived from plants as raw materials that were highly safe for human bodies. Issues related to the costs of such raw materials were resolved by obtaining ceramide-related substances from brewers' grains with reference to Patent Document 4 (glycosphingolipid derivatives).

According to Patent Document 4, a method for sufficiently drying brewers' grains in order to extract the targeted ceramide-related substances derived from plants in an efficient manner and at low cost is provided. With such method, polar solvents for extraction can be efficiently immersed in brewers' grains, Furthermore, mixing of moisture contained by the brewers' grains in polar solvents can be suppressed. Moreover, Patent Document 4 provides a method for recycling the obtained extracts for polar solvents for extraction. Through such method, the quantity of polar solvents necessary for extraction can be reduced to about 1/10^{th}. As known art for obtaining ceramide-related substances from brewers' grains, known art according to Patent Documents 5 and 6 has been recognized, in addition thereto. It is thought that use of the method in Patent Document 4 would be preferable in terms of reduction of manufacturing costs.

The present inventors repeatedly conducted intensive research using glycosphingolipid derivatives obtained from the brewers' grains mentioned above. As a result, the present inventors discovered the fact that such glycosphingolipid derivatives showed strong cytotoxic activation only against specific tumor cells, and were almost inactive against normal cells. Most of the ceramide-related substances that had been known thus far targeted all tumor cells, such as α-Galactosyl ceramide (hereinafter referred to as "αGalCer"), and showed strong cytotoxic activation against normal cells as well (Non-Patent Document 2). The glycosphingolipid derivatives of the present invention showing cytotoxic activation that is selective with regard to cells have not been reported thus far, as far as the present inventors are aware.

Furthermore, the present inventors discovered the fact that the glycosphingolipid derivatives obtained through the brewers' grains mentioned above had immunostimulatory activity that activated Natural Killer T cells (hereinafter referred to as "NKT cells") not accompanied by induction of liver damage, even when high concentrations of the glycosphingolipid derivatives were administered. It has been reported thus far that aGalCer had the same type of immunostimulatory activity and apoptosis induction (Non-Patent Document 3). However, in regards to administration of αGalCer, due to its strong cytotoxic activation, side-effects such as liver damage and the like have constituted a major issue. Thus, it can be thought that the glycosphingolipid derivatives of the present invention with fewer side-effects are more effective.

The present invention has been completed based on the related discoveries, and provides the following (A) through (K).

(A) A medical composition, which comprises more than one glycosphingolipid derivative represented by the following formula (1) as an active ingredient. 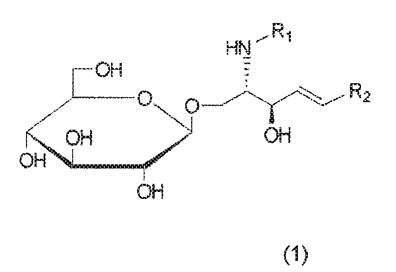 wherein R₁ represents a residue in which monocarbonic acid is combined with a carboxyl group in an acid amide combination, and R₂ is (CH₂)ₓ₁ CH=CH(CH₂)ₓ₂ CH₃ (where X₁ and X₂ corresponds to 0 or to a natural number and X₁ + X₂ = 10).

(B) A medical composition, which comprises more than one glycosphingolipid derivative represented by the following formula (2) as an active ingredient. 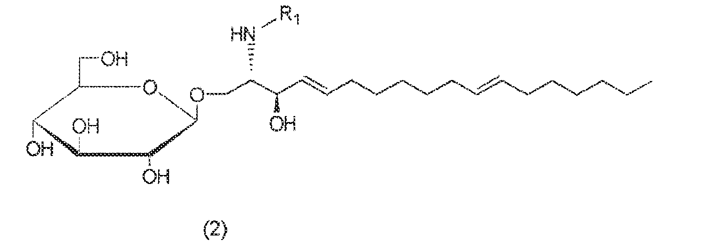 wherein R₁ represents a residue in which monocarbonic acid is combined with a carboxyl group in an acid amide combination.

(C) The present invention provides a medical composition, which comprises more than one glycosphingolipid derivative represented by the following formula (3) as an active ingredient. 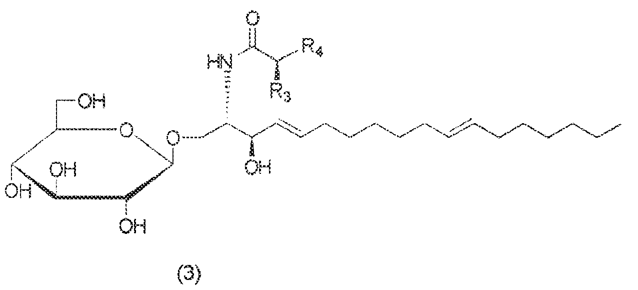 wherein R₃ represents H or OH and R₄ corresponds to either the following (a) or (b):
(a) when R₃ is H, R₄ is (CH₂)₁₃ CH₃ or (CH₂)₆CH=CHCH₂CH=CH(CH₂)₄ CH₃; and
(b) when R₃ is OH, R₄ is (CH₂)_{Y} CH₃ (where Y is an integer from 13 - 21) or (CH₂)_{Z1} CH=CH(CH₂)_{Z2} CH₃. (where Z₁ and Z₂ corresponds to 0 or to a natural number and Z₁ + Z₂ is 19).

(D) The present invention provides the medical composition, wherein (b) mentioned above is defined as follows:
(b) When R₃ is OH, R₄ is (CH₂)_{Y} CH₃ (where Y is an integer from 13 - 21) or (CH₂)₁₂ CH=CH(CH₂)₇CH₃.

(E) The present invention provides the medical composition, wherein the glycosphingolipid derivatives are natural glycosphingolipid derivatives.

(F) The present invention provides the medical composition, wherein the glycosphingolipid derivatives are extracted from brewers' grains obtained through a process for manufacturing of beers and the like.

(G) The present invention provides the medical composition, wherein the medical composition is used for an antitumor agent.

(H)The present invention provides the medical composition, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above formula (1) as an active ingredient.

(I) The present invention provides the medical composition, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above formula (2) as an active ingredient.

(J) The present invention provides the medical composition, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above Formula (3) as an active ingredient.

(K) The present invention provides the medical composition, such medical composition being used for an immunostimulating agent.

### [Advantageous Effects of the Invention]

The compositions of the present invention may be medical compositions as antitumor agents with strong cytotoxic activation targeting only specific tumor cells that have remarkably low toxicity for normal cells (that is to say, that have fewer side-effects).

Additionally, the compositions of the present invention may be medical compositions as immunostimulating agents with fewer side-effects that do not result in cytoduction, such as liver injury and the like.

Furthermore, the compositions of the present invention may be low-cost medical compositions produced from raw materials that are highly safe for human bodies.

### [Detailed Description of the Preferred Embodiments]

Hereinafter, embodiments of the present invention will be described. In addition, the present invention is not restricted by the embodiments at all. That is to say, the present invention can be implemented in various forms, without deviating from the purpose thereof.

The term "glycosphingolipid derivative(s)" used in the present invention refers to a composition that has a basic structure of glycosylceramide as a glycosphingolipid, and it also refers to a group of substances in which part of the structure within the molecules of such glycosylceramide, such as the number of carbons in a fatty acid, the locations of double bonds, hydroxy addition, or the like, has been changed. That is to say, such composition is represented by any of Formula (1), Formula (2), or Formula (3) mentioned above.

Herein, R₁ in Formula (1) represents a residue in which monocarbonic acid is combined with a carboxyl group in an acid amide combination. The carbon chain number for such monocarbonic acid is any integer from 2 through 30. Any integer from 16 through 24 is preferable. Additionally, in case that the carbon chain number of R₁ falls within the scope of the integers from 4 through 30, one double bond may exist in such carbon chain. If so, the location of such double bond is not particularly limited as long as the location is within the carbon chain mentioned above. Additionally, R₂ has the carbon chain number 13, and one double bond exists in such carbon chain. As long as the location of such carbon chain falls within the scope represented by C(CH₂)_{X1} CH=CH(CH₂)_{X2} CH₃(herein, X₁ and X₂ correspond to 0 or to a natural number, and X₁ + X₂ = 10), any location is acceptable. A location shown by Formula (2) or Formula (3), that is to say, a location represented by (CH₂)₅ CH=CH(CH₂)₅CH₃, is preferable.

Furthermore, R₃ of Formula (3) is hydrogen ("H") or a hydroxyl group ("OH"). The structures of R₄ differ in each case. That is to say, in case that R₃ is H, a residue exists in which palmitic acid or linoleic acid is combined with a carboxyl group in an acid amide combination. This is shown by Formula (4) or Formula (5) as below.

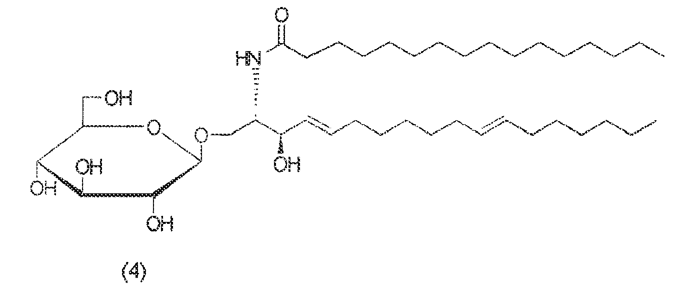

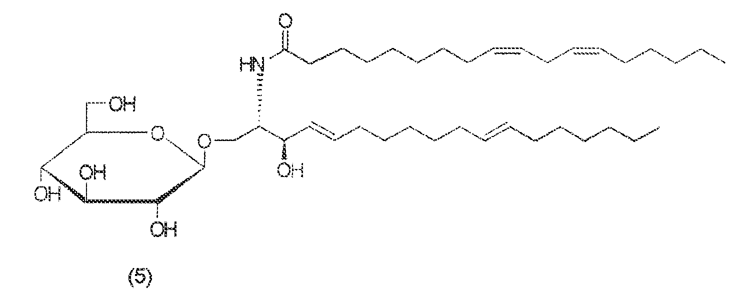

In case that R₃ is OH, the monocarboxylic acid residue is a saturated fatty acid residue with a carbon chain number of 16 through 24, or an unsaturated fatty acid residue with a carbon chain number of 24 in which a double bond is included in such carbon chain.

That it to say, the former case is shown by Formula (6) as below. 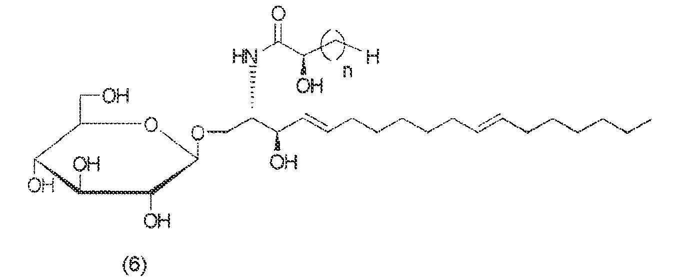 Herein, "n" corresponds to any one of the integers from 14 through 22.

Additionally, the latter case is shown by Formula (7) as below. 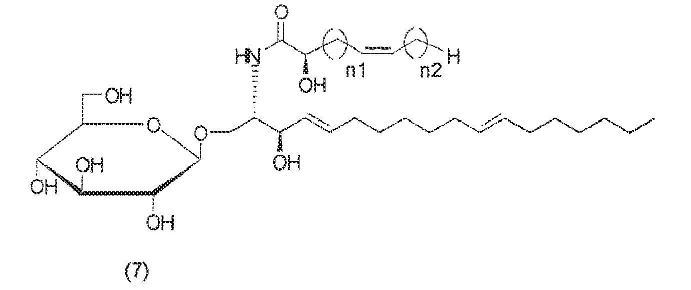 Herein, "n1" and "n2" correspond to 0 or a natural number and correspond to "n1 + n2 = 20." In regards to double bonds based on Formula (7), a structure with cis-position double bonds based on C15 - represented by Formula (8) as below is preferable.

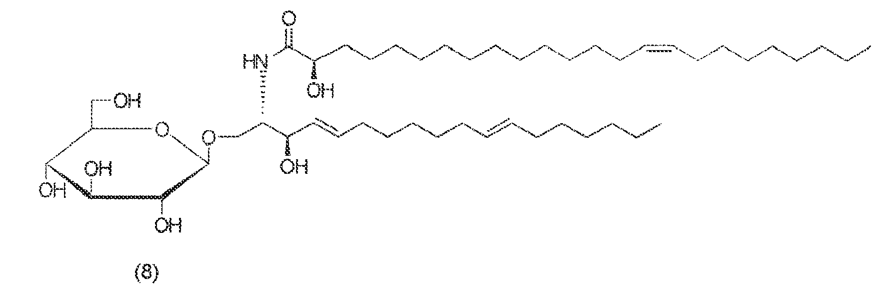

The term "medical compositions" of the present invention refers to items broadly used for medical drugs or raw materials used therefor, such as antitumor agents, immunostimulating agents, apoptosis-inducing drugs, and the like. In particular, the present invention has the two effects of antitumor activity and immunostimulatory activity described below. Thus, the medical compositions that are antitumor agents and immunostimulating agents are preferable.

<Extraction of glycosphingolipid derivatives >

Glycosphingolipid derivatives shown based on the aforementioned formula (1), formula (2), or formula (3) of the present invention may be produced based on chemical synthesis. However, one of the purposes of the present invention is to produce items made from raw materials that are safe for human bodies. With due consideration given thereto, it is difficult to say that production of items based on chemical synthesis is sufficiently safe as of the present time. Therefore, glycosphingolipid derivatives extracted from natural raw materials are preferable. Furthermore, glycosphingolipid derivatives extracted from plant tissues are further preferable. From among glycosphingolipid derivatives extracted from plant tissues, glycosphingolipid derivatives extracted from brewers' grains acquired in the process for manufacturing of beers and the like are particularly preferable. This is because brewers' grains contain great quantities of glycosphingolipids of the present invention, as shown in the first embodiment below.

The term "beer(s) and the like" refers to beers or types of alcohol similar to beer. The term "types of alcohol similar to beer" herein refers to alcohol beverages with appearances and flavors similar to those of beers. For instance, one such example is low-malt beer.

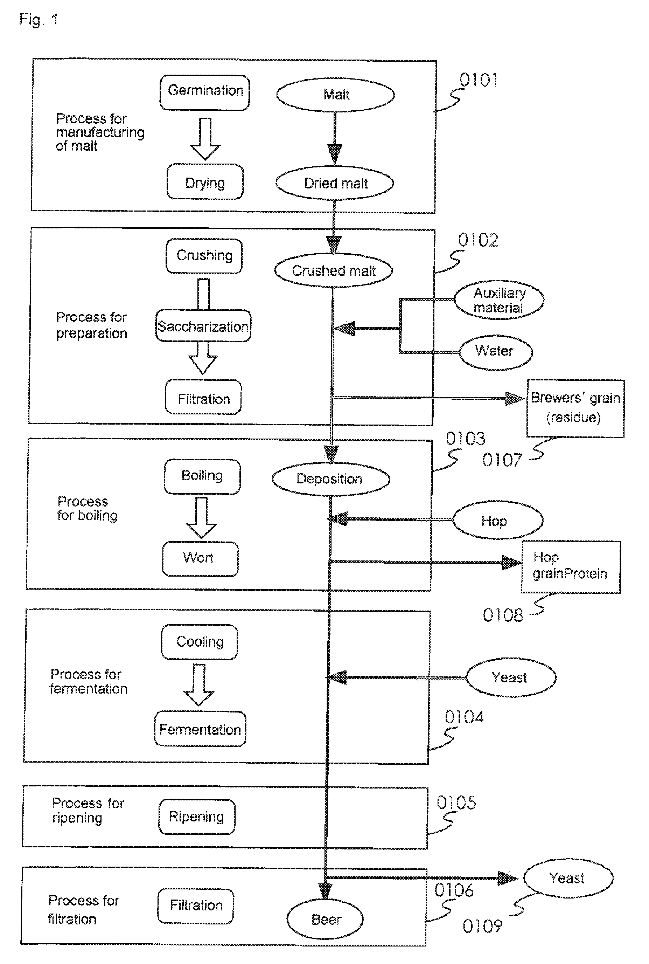

Explanations are given with reference to examples concerning "the process for manufacturing of beers" using Fig. 1. As shown in this Fig., the initial process in the general process for manufacturing of beers corresponds to a process for manufacturing malt (0101). In this process, after barley has germinated, the resultant is dried up through hot air or the like, its growth ceases, and dried malt is obtained. Subsequently, the process moves on to the process for preparation (0102). In this process, the aforementioned malt is crushed into pieces. Warm water, corn starch, and the like as sub-raw-materials are added to the resultant, and due to operation of malt enzymes, saccharification of starch is undertaken. After this process, wort is filtered. Residue remaining after such filtration corresponds to brewers' grains (0107). Next, the process for boiling (0103) is undertaken. In this process, the aforementioned wort is boiled and hops as bittering agents are added thereto. Proteins and hop grains (0108) after boiling are precipitated as grouts and removed. Subsequently, a process for fermentation (0104) is undertaken. In this process, the aforementioned wort after boiling is cooled down, yeast is added thereto, and the resultant is fermented approximately for 1 to 2 weeks (depending on fermentation temperature). Subsequently, the process moves on to the process for ripening (0105). In this process, the wort after fermentation is cooled to close to 0°C, and fermentation is suppressed. Herein, carbon dioxide gas is accumulated and the taste becomes mild. The final process is the process for filtration (0106), In this process, yeast (0109) is filtered through liquids after the process for ripening, and draft beer is acquired. After filtration, heat-sterilized beer becomes normal beer. After the processes mentioned above has been undertaken, general beers are manufactured. In addition, raw materials used for types of alcohol similar to beer differ from those used for beers regulated under the liquor tax law. However, the basis for the manufacturing process is almost the same as that used for beers mentioned above.

The term "brewers' grain" (0107) refers to residues acquired after the process for preparation in the process for manufacturing of beers and the like mentioned above. Thus, normally, brewers' grains are composed of malts as well as starch from rice, corn, potatoes, and the like, which are used as sub-raw-materials, grains that contain sugars, or strained potatoes. Of course, residues that are 100% composed of malts are acceptable. Also, proteins and hop grains (0108) resulting after the process for boiling (0103) and yeast (0109) acquired after the process for filtration may be added as a parts of the aforementioned brewers' grains. This is because, in relation to hop grains, yeast, and the like, there is a possibility that mixture of glycosphingolipid derivatives that have been changed to pomaces could occur, or that such derivatives could undergo the process for fermentation, without such derivatives being collected from brewers' grains.

Any publicly known methods may be used as methods for extracting glycosphingolipid derivatives from natural raw materials of animal tissues, plant tissues, and the like. In particular, in regards to a method for extracting glycosphingolipid derivatives from brewers' grains, it is convenient to do so in conformance with the techniques of Patent Document 4 or 6.

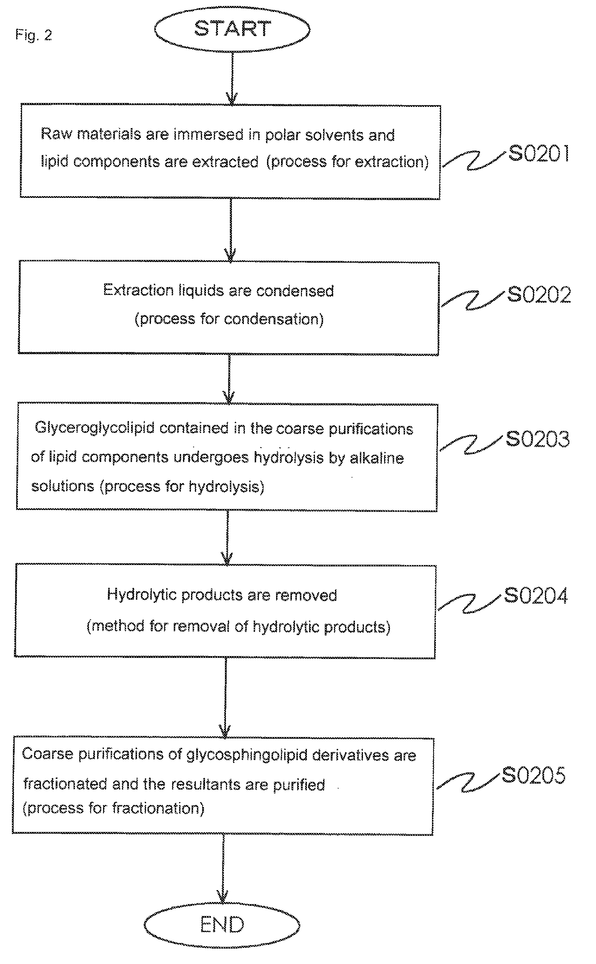

Explanations are provided with reference to diagrammatic representation in regards to the most general method for extracting glycosphingolipid derivatives from natural raw materials in Fig. 2.

First, raw materials are immersed in polar solvents and lipid components are extracted (S0201: process for extraction). And extraction liquids are condensed (S0202: process for condensation). Through the aforementioned treatment, coarse purifications contained in raw materials can be obtained.

At this point, the term "polar solvent(s)" refers to solvents that are composed of polar molecules with electric charge bias. For instance, examples of the aforementioned solvents are polar organic solvents, such as lower alcohol, benzene, or toluene and the like, water, or mixed liquids based on combinations thereof, Types of polar solvents and combinations thereof are not especially restricted. However, when glycosphingolipid derivatives acquired through the present invention are used for human beings and other animals, ethanol with remarkably low toxicity or a mixed liquid of ethanol and water is preferable. Moreover, when extraction efficiency is considered, use of ethanol alone is preferable.

Subsequently, alkaline solutions are added to coarse purifications that are composed of lipid components acquired through the aforementioned method. And glyceroglycolipid contained in the coarse purifications of such lipid components undergoes hydrolysis by alkaline solutions (S0203: process for hydrolysis). Thereafter, hydrolytic products are removed (S0204: method for removal of hydrolytic products). Due to such operations, coarse purifications of glycosphingolipid derivatives can be obtained.

The term "alkaline solutions" refers to solutions in which alkaline materials are dissolved by polar solvents. For instance, alkaline solutions of the present invention can use sodium hydroxide solutions or potassium hydroxide solutions. The alkali concentration of alkaline solutions and added contents may be acceptable if alkali quantity in the solvents has a saponification value that allows hydrolysis of glyceroglycolipids contained in the coarse purifications of lipid components mentioned above.

In order to accelerate hydrolysis reaction, mixed liquids of coarse purifications of lipid components and alkaline solutions may be heated. A heating temperature of 30°C to 50°C is preferable. The time necessary for hydrolysis reaction depends on the temperature. That is to say, the lower the temperature is, the longer the necessary time. For instance, adjustments such as "2 hours at 37°C" or "1 hour at 50°C" may be made accordingly.

After hydrolysis processing, mixed liquids composed of hydrophobic solvents, such as chloroform, and hydrophilic solvents, such as water or methanol, and the like, are added and mixed. The resultant is allowed to stand still and divided into 2 layers. When chloroform as a hydrophobic solvent is used, chloroform layer from a bottom layer that contains the targeted glycosphingolipid is collected. Due to this, fatty acid and glycerin as degraded products that have been transferred to the water (hydrophobic solvent) layer are removed. Chloroform is removed from the solvents of the chloroform layer via an air-drying method and the like. Through this method, coarse purifications of glycosphingolipid derivatives can be obtained.

As needed, the coarse purifications of glycosphingolipid derivatives mentioned above may be further purified. In such case, it is possible to do so based on fractionation of the coarse purifications of glycosphingolipid derivatives that have been obtained (S0205: process for fractionation).

A method for fractionation may be in conformance with publicly known technologies. For instance, there exist methods for fractionation and elution based on methods for thin layer chromatography (TLC), adsorption chromatography, partition chromatography, high-performance liquid chromatography (HPLC), and the like. A specific example of chromatography is column chromatography. In regards to such column chromatography, the coarse purifications of glycosphingolipid derivatives are loaded on a stationary silica gel phase or the like. Thereafter, elution takes place through solutions in which hydrophobic solvents such as chloroform, hydrophilic solvents such as methanol, and solvents of a plurality thereof are mixed based on the appropriate content ratio.

Upon elution, composition of solutions and time for elution are properly adjusted. Due to differences in solubility and ion bonding force in regards to solvents of glycosphingolipid derivatives, furthermore, as needed, via repetition of the same operations several times, the targeted glycosphingolipid derivatives can be separated and purified. In addition, detailed explanations of a method for preparation for the glycosphingolipid derivatives of the present invention are given in the first embodiment.

In addition to the aforementioned processes, the following processes may be acceptable: "a process for drying" that removes moisture contained in brewers' grains prior to the "process for immersion" described in the Patent Document 4 and "a process for recycling" that repeats recycling of extraction liquids obtained through removal of brewers' grains after the process for immersion as the polar solvent through the process for immersion with a given frequency. To use the aforementioned processes can reduce costs of raw materials and contribute to the purposes of the present invention. A method for dying and a method for recycling may conform to the technologies stated in Patent Document 4.

It is not necessary that the glycosphingolipid derivatives obtained through the methods mentioned above be purified to result in a single substance. For instance, in the case of brewers' grains as raw materials, after extraction of polar solvents, the glycosphingolipid derivatives may be used as the coarse purifications of the glycosphingolipid derivatives concerning which only alkali decomposition processing takes place. Additionally, even when such coarse purifications are further purified, a mixture in which a several types of derivatives that normally have different numbers of carbons are mixed can be obtained in many cases. It is not necessary to separate and purify such derivatives that normally have different numbers of carbons. This is because such mixture contains the glycosphingolipid derivatives of the present invention. And as shown in the second through the sixth embodiments, as a matter of fact, the effects of the present invention are sufficiently fulfilled with such mixture.

< Antitumor activity of glycosphingolipid derivatives>

Glycosphingolipid derivatives shown in Formula (1) through Formula (3) of the present invention have strong cytotoxic activation against cultured cells derived from tumor cells in the same manner as aGalCer and the like, as described in the second embodiment. What is of interest herein is the fact that such glycosphingolipid derivatives show cytotoxic activation against the specific tumor cells alone, within the scope of a given applied dose, and hardly show the same in regards to other tumor cells. Furthermore, an important point is the fact that glycosphingolipid derivatives hardly show cytotoxic activation against normal cells within the scope of a given applied dose. This shows that such glycosphingolipid derivatives have fewer side-effects. As such, no report has been made in regards to glycosphingolipid derivatives that selectively show cytotoxic activation against specific tumor cells alone, as far as the inventor is aware. Thus, such glycosphingolipid derivatives have fewer side-effects in regards to normal cells, and attack only specific tumors as targets. In this regard, the glycosphingolipid derivatives are useful for medical compositions, and in particular, for antitumor agents.

Herein, the term "specific tumor cell(s)," for example, refers to colon cancer cells, liver cancer cells, skin cancer cells, lung adenocarcinoma cells, leukemia cells, and the like. On the other hand, tumor cells in which the glycosphingolipid derivatives do not show cytotoxic activation -- that is to say, tumor cells in which the glycosphingolipid derivatives do not show antitumor activity -- include lymphoma cells, gastric cancer cells, pancreas cells, squamous cell cancer of the lung, and the like, for example. Additionally, the "given applied doses" above are considered in light of types of targeted tumors, the situations of patients as described below, and the like. In addition, detailed explanations of antitumor activity regarding the glycosphingolipid derivatives of the present invention are given with reference to the second embodiment.

< Immunostimulatory activity of glycosphingolipid derivatives >

Glycosphingolipids such as αGalcer and the like, are known to activate NKT cells accompanying apoptosis induction (Non-Patent Documents 4-6). The glycosphingolipid derivatives shown through Formulas (1) through (3) of the present invention have immunostimulatory activity that activates NKT cells accompanying apoptosis induction in the same manner as with αGalcer as described in the third and sixth embodiments. However, in regards to points of difference regarding immunostimulatory activity of glycosphingolipid derivatives as compared with αGalcer, while glycosphingolipid derivatives have immunostimulatory activity as described in the third embodiment, hardly any increase in the INF - γ level in the blood is recognized, as described in the fifth embodiment. Furthermore, even in regards to the highly concentrated dose described in the fourth embodiment, side-effects of liver damage hardly occur. That is to say, the glycosphingolipid derivatives of the present invention are derived from the same type of glycosphingolipids, such as αGalcer and the like, which have been known in the past. However, it can be thought that mechanisms of action of and effects given to living organisms by the glycosphingolipid derivatives of the present invention differ. As mentioned above, glycosphingolipid derivatives impose fewer side-effects on liver cells and activate NKT cells. Thus, glycosphingolipid derivatives are useful for medical compositions, and in particular, for immunostimulating agents.

<Method for use as medical compositions>

In regards to medical compositions that have glycosphingolipid derivatives of the present invention as active ingredients, the glycosphingolipid derivatives themselves or a resultant in which such glycosphingolipid derivatives are prepared with an appropriate carrier as a medicinal preparation can be administered to human beings or animals.

Methods for administration are not in particular restricted as far as administration channel suitable for targeted purposes is concerned. For instance, in the case of human beings, local administration via an injection or the like, intravascular administration in veins or arteries, intraperitoneal administration, intrathoracic administration, intramuscular delivery, rectal administration, subcutaneous administration, percutaneous absorption, oral administration, sublingual administration, and the like are possible. Additionally, in the case of animals, local administration via an injection or the like, intravascular administration in veins or arteries, intraperitoneal dministration, subcutaneous administration, and the like are possible.

Forms of medicinal preparation may be selected based on methods of administration, purposes of administration, and the like as necessary. For instance, oral forms include pill forms, encapsulated formulations, fine grain agents, powdered drugs, pastilles, dry syrups, and the like. Parenteral agents include injectable solutions, suspension agents, emulsifying agents, ointments, suppository forms, patches, and the like. In regards to carriers used for medicinal preparations, additives permissible in the course of medicine manufacture may be selected according to methods of administration, purposes of administration, and the like as necessary. Additives include diluents of solvents and solubilizing agents, pH adjusters, viscosifiers, tonicity agents, diluents, binders, lubricants, stabilizers, preservatives, antioxidants, surfactants, and the like.

The dose quantity for glycosphingolipid derivatives as active ingredients of the present invention may be determined so that a certain such quantity will not be exceeded in a consecutive or intermittent manner, in light of results of animal experiments and individual situations. Specific dose quantities differ based on methods of administration, situations of patients, and the like. Herein, "situations" refer to age, sex, weight, diet, dose duration, companion drugs, drug susceptibility, degree of disease, and the like. Moderate amounts, quantity of dose, and frequency of dose must be determined based on tests to determine moderate amounts by experts in accordance with the policies mentioned above.

The present invention will be explained hereinafter based on the following embodiments in a more detailed manner. However, the embodiments provide only exemplifications of the present invention, and they do not in any way restrict the scope of the present invention.

### First embodiment

<Method for preparation of glycosphingolipid derivatives of the present invention>

The embodiment related to extraction of glycosphingolipid derivatives derived from brewers' grains mentioned above is explained as below.

Glycosphingolipid derivatives as chemical compounds of the present invention were prepared under conditions in which brewers' grains were used as raw materials. First, a method for purifying glycosphingolipid derivatives as ceramide-related substances from brewers' grains in a coarse manner was conducted in accordance with the method of first embodiment including the process for condensation described in Patent Document 4. 2 g of a residue of coarse glycosphingolipid derivatives as described in Patent Document 4 was dissolved in 4 ml of chloroform. Subsequently, 4 ml of 0.6 N sodium hydroxide as a solvent of methanol was added thereto and mixed therewith. Thereafter, the resultant was kept warm for 30 minutes at 50 °C. Through such operation, glyceroglycolipid, which was mixed with the coarse glycosphingolipid derivatives mentioned above, experienced alkali hydrolysis. Next, 2.6 ml of 1N hydrochloric acid and 1 ml of water were added and neutralization took place. Thereafter, the resultant was left for 3 hours at room temperature. As a result, layer detachment took place. From among detached layers, a methanol layer containing glyceroglycolipid that experienced alkali hydrolysis was removed, and a chloroform layer containing glycosphingolipid derivatives was obtained.

Subsequently, solutions of the chloroform layer obtained through the process mentioned above were each placed in an open silica gel column (Merck, 1.5 x 30 cmID). Thereafter, the resultant was eluted via a mixed liquid of chloroform, methanol, and water (content ratio = 65:15:2). Mainly, nonpolar lipid fractions composed of fatty acid, fatty alcohol, and sterol were separated and removed after 60 minutes of elution, and sterol ether fractions were separated and removed after 110 minutes of elution. Fractions of glycosphingolipid derivatives were obtained after 120 minutes of elution. Through this process, coarse purification of glycosphingolipid derivatives was conducted.

Next, the fractions of glycosphingolipid derivatives acquired through the process mentioned above were placed in a high-performance liquid chromatography silica filler column (Develosil, Develosil60-3, 8 mm x 250 nmID). In regards to elution from column, a mixed liquid of chloroform and methanol (content ratio = 87:3) was made to flow at a flow speed rate of 2.5 ml/minute at 40°C. And purified glycosphingolipid derivatives were obtained during 20 minutes of elution. In addition, all procedures that were not particularly specified concerning the experiments mentioned above were conducted at room temperature (about 28°C).

### <Methods for analysis of purified glycosphingolipid derivatives>

Spectral data regarding the glycosphingolipid derivatives as final coarse purifications mentioned above are given below.

(¹H)
NMR: 500 MH₂, CDCl₃, and 23.9°C
δ (ppm) 5.35 (m), 5.35 (m), 5.35 (m), 5.35 (m), 4.27 (d, J = 7.9), 4.24 (d, J = 7.4), 4.08 (m), 4.02, 3.96 (m), 3.80 (dd, J = 10.5, 3.8), 3.79 (dd, J=3.8, 10.0), 3.66 (m), 3.35 (m), 3.30 (m), 3.29 (m), 3.28 (m), 2.06 - 2.00,1.28 - 1.401.28 (m), 1.28 (m), 1.28 (m), 1.28 (m), 1.28 (m), 1.28 (m), 1.24 - 1.24, 1.24 - 1.24, 0.89 (t), 0.89 (t).

(¹³H)
NMR: 500 MH₂, CDCl₃, and 23.9°C
δ (ppm) 1.77.1, 130.8, or 130.7, 130.8 or 130.7, 130.1 or 130.8, 130.1 or 130.8, 104.7, 77.9, 77.9, 75.5, 72.9, 71.6, 71.6, 89.1, 62.6, 54.6, 35.9, 33.7, 32.9, 31.0 - 30.3, 31.0 - 30.3, 31.0 - 30.1, 26.1, 23.8, 23.8, and 14.5.

As a result of such spectral data, it was revealed that the glycosphingolipid derivatives as the final coarse purifications corresponded to glycosphingolipid derivatives
in which R₃ and R₄ of formula (3) have the structures shown in Table 1.

**[Table 1]**

| R₃ | R₄ | Percentage (%) |
|---|---|---|
| H | (CH₂)₁₃ CH₃ | 4.6±0.0 |
| H | (CH₂)₆ CH=CHCH₂CH= CH(CH₂)₄ CH₃ | 10.4±0.1 |
| OH | (CH₂)₁₃ CH₃ | 10.2±0.1 |
| OH | (CH₂)₁₅ CH₃ | 11.0±0.1 |
| OH | (CH₂)₁₇ CH₃ | 27.1±0.1 |
| OH | (CH₂)₁₉ CH₃ | 9.5±0.0 |
| OH | (CH₂)₂₁ CH₃ | 10,0±0.1 |
| OH | (CH₂)₁₂ CH= CH(CH₂)₇ CH₃ | 17.1±0.2 |

### Second embodiment

<Cell selection antitumor activity regarding the glycosphingolipid derivatives of the present invention>

### (Object)

Cytotoxic activation is revealed in which the glycosphingolipid derivatives of the present invention influence cultured cells.

### (Method for experiment)

A method for experiment is explained hereinafter. In addition, according to the embodiment, all equipment, reagents, water, and the like used for cell culture underwent a sterilization process in principle, unless otherwise noted in particular. Operations were conducted subject to the conditions of a clean bench or a bioclean room.

Type of culture cell line:
In this embodiment, the following culture cell lines derived from human tumor cells and normal culture cell lines derived from the same histogenous cells as available tumor cells were used: Hep-G2 (liver cancer cell), CS-HC (normal liver cell), A431 (squamous cell cancer), TIG (normal skin cells), A549 (lung adenocarcinoma cell), W138 (normal lung cell), W138 VBA sub 2RA (normal lung cell), OUMS36 (normal fibroblast), MOLT-4 (T-cell leukemia cell), COLO201 (colon cancer cell), Raji (lymphoma cell), MIA Paca (pancreas cancer cell), VMRC-LCP (squamous cell carcinoma cells of the lung), and KATO-3 (gastric cancer cell).

### Culture reagent:

In order to culture the cells mentioned above, the following culture media were used.
· Used for Hep-G2, A431, A549, and OUMS38: Dulbecco's Modified Eagle Medium (DMEM: NACALAI TESQUE, INC.)/10% Fetal Bovine Serum (FSC: Fetal Calf Serum; Sigma)
· Used for MOLT-4, COLO201, Raji, MIA Paca: RPMI1640 (Sigrua)/10% Fetal Bovine Serum (FSC: Fetal Calf Serum;Sigma)
· Used for TIG, W138, and VMRC-LCP: Eagle's minimum essential medium (E-MEM: Gibco)/10% FCS (Sigma)
· Used for KATOIII: McCoy's 5a (Sigma)/10% FCS (Sigma)
· Used for W138 VBA sub2RA: E-MEM + NEAA (nonessential amino acid: Gibco) + pyruvic acid (Gibco)/10% FBS (Sigma)
· Used for CS-HC: CS-C complete medium (Dainippon Pharmaceutical Co., Ltd.)/10% FCS (Sigma)

### Method for culture and addition of glycosphingolipid derivatives

Conditions for culture of cultured cells and conditions for addition of glycosphingolipid derivatives are explained. Using a 96-well plate, established cell lines of 3.0 x 10⁵ cells of the cultured cells listed above were disseminated at 100 µl/well x 5 wells of cultures suitable for the respective cell lines. Such plates were cultured at 37°C, under 5% CO₂ concentration for 24 hours. Thereafter, mixed liquids of glycosphingolipid derivatives listed in Table 1 that were purified in the first embodiment were added to wells at different concentrations. Concentrations for added glycosphingolipid derivatives were each of any one of the following 5 categories: additive-free, 50 µM, 100 µM, 150 µM, or 200 µM; or they were each of any one of the following 5 categories: additive-free, 25 µM, 50 µM, 75 µM, or 100 µM. Preparations for concentrations were conducted based on a phosphoric acid buffer solution including 0.8% Tween80. All samples used were filtered with a 0.22-µM filter and were sterilized prior to addition. After addition, incubation was conducted again at 37°C under a 5% CO₂ concentration for 24 hours. In addition, exchange of culture media within the wells was not performed during the above culturing.

### Measurement of the number of living cells (MTS assay)

After the incubation mentioned above, CellTiter 96^{(R)} A Queous Non-Radioactive Cell Proliferation Assay (Promega) as a reagent used in the detection of apoptosis was added to each well in accordance with the attached protocol. Incubation was conducted at 37°C under a 5% CO₂ concentration for a period of 1 to 4 hours. In regards to living cells, tetrazolium salt MTS was reduced during use of the reagent of such kit to cause chromogenic formazan products that can be detected at 490 nm. Thus, after incubation, it is possible to discover the percentage of cells in which apoptosis has been induced by the glycosphingolipid derivatives of the present invention based on a measurement of absorbance at 490 nm. In regards to the measurement, an unprocessed control was cultured concurrently in advance. And it is possible to obtain the percentage of living cells in well of living cells by making comparisons with absorbance values obtained through the wells processed by the glycosphingolipid derivatives and control values. In regards to unprocessed control, an additive-free sample of the glycosphingolipid derivatives mentioned above may be used. Alternatively, another item that has been separately prepared may be acceptable. Via performance of measurement based on several concentrations of the glycosphingolipid derivatives, it was possible to obtain survival curve fluctuation in regards to added concentrations. In addition, in this embodiment, records of the percentages of living cells were determined more than 5 times under the same concentration for each cultured cell. The average values thereof corresponded to the percentage of living cells of the corresponding concentration.

### (Results)

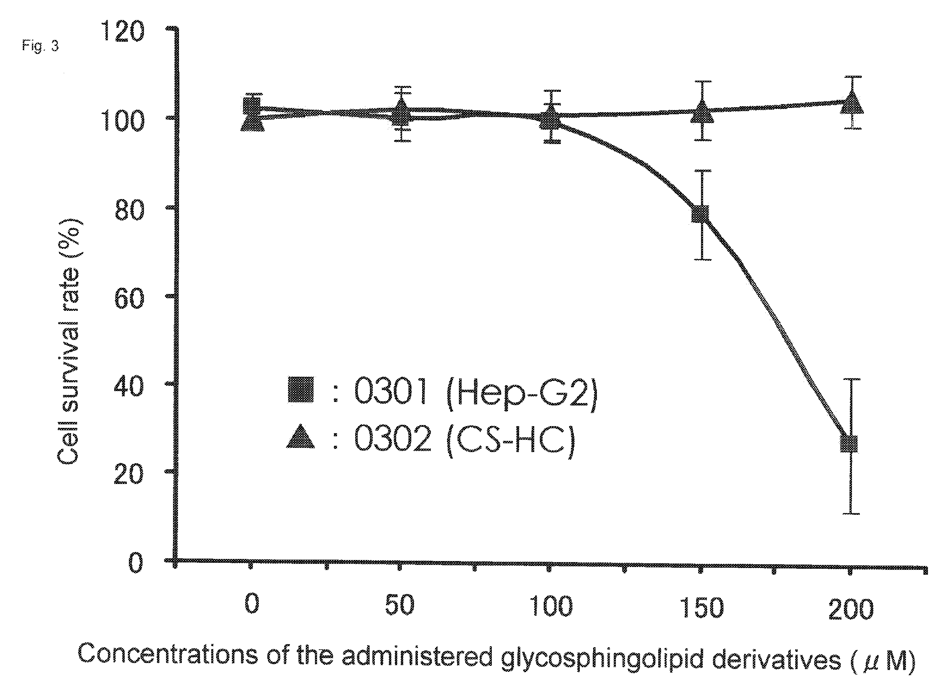

Measured results for the percentage of living cells for each cultured cell regarding the experiments mentioned above and the survival curves based thereupon are shown in Fig. 3 through Fig. 12. Graphs of all figures show concentrations of the added glycosphingolipid derivatives (µM) on the horizontal axis and the percentage of living cells (%) on the vertical axis. Cells shown in each figures are as follows.
Fig. 3: Hep-G2 (0301), CS-HC (0302)
Fig. 4:A431 (0401), TIG
Fig. 5: A549 (0501), WI38 (0502), WI38 VBAsub R A2 (0503)
Fig. 6:MOLT-4
Fig. 7:COLO201
Fig. 8:OMUS-36
Fig. 9:Raji
Fig. 10: MIA Paca
Fig. 11:VMRC-LCP
Fig. 12:KATO-3

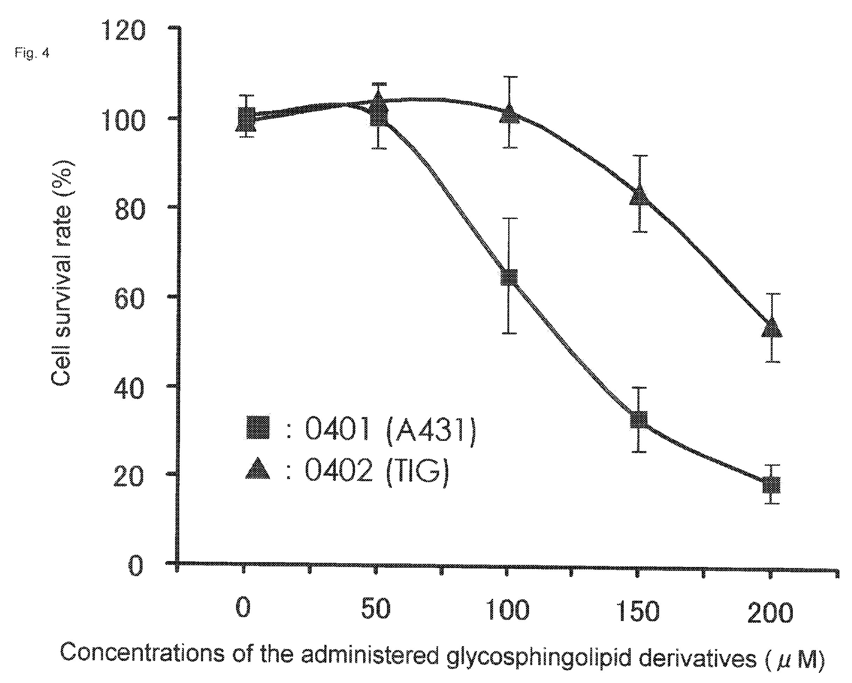

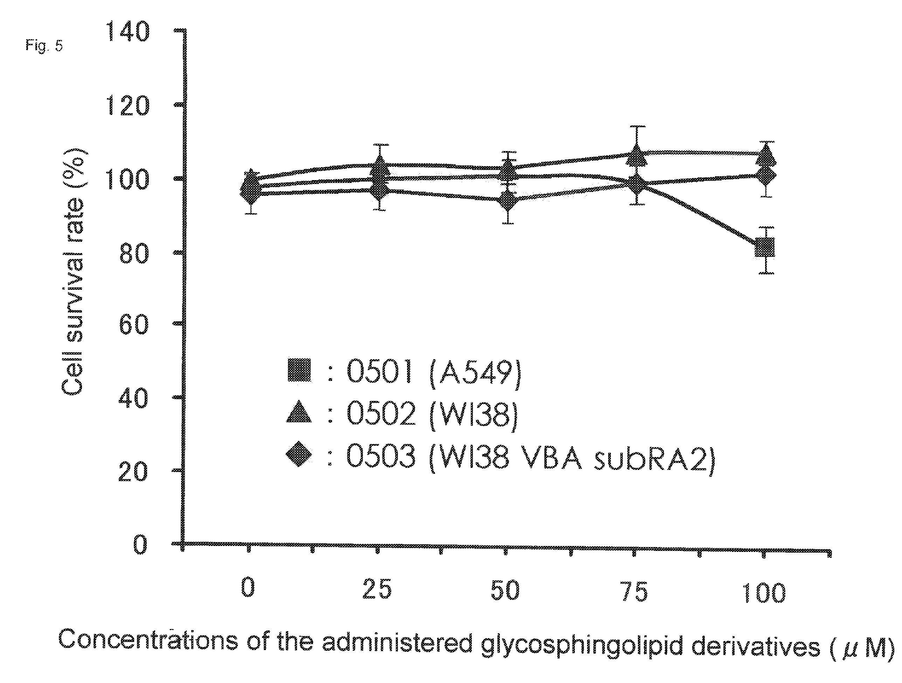

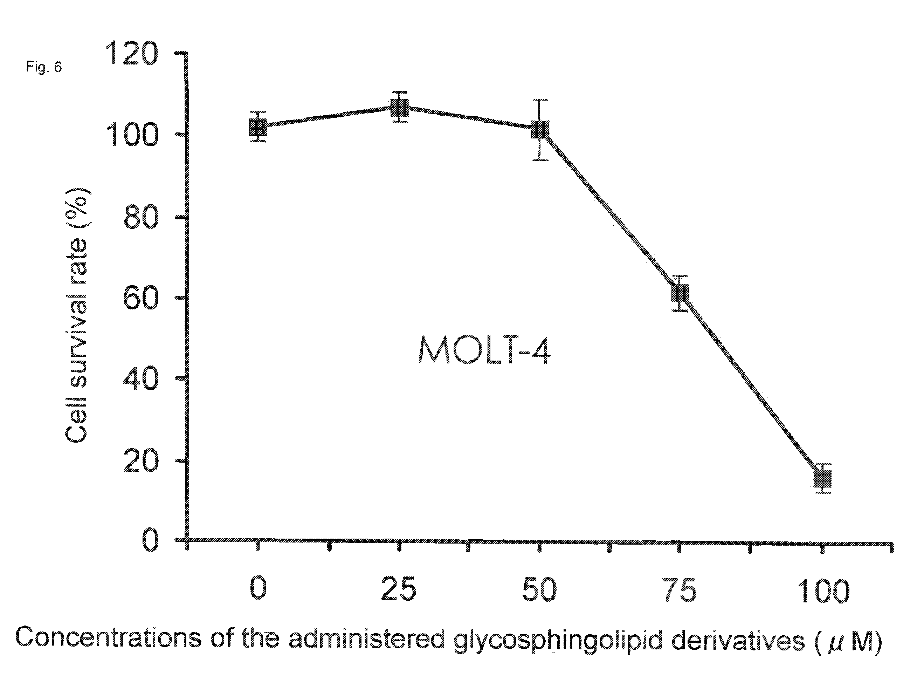

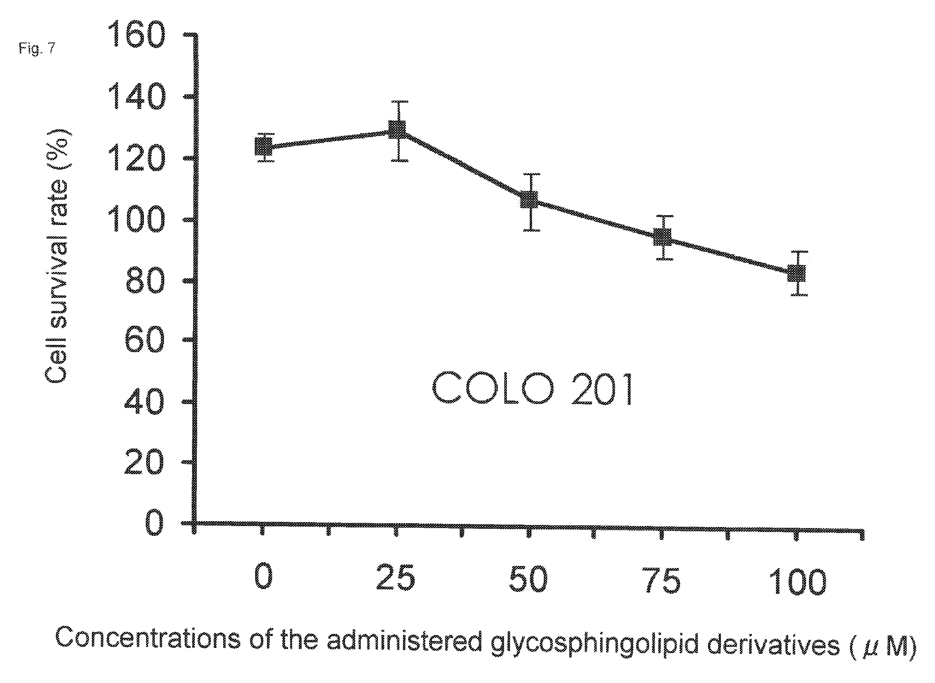

As a result of survival curves, it was revealed that the glycosphingolipid derivatives of the present invention showed remarkable cytotoxic activation under the concentrations of 100 µM or 200 µM in regards to Hep-G2 liver cancer cells (Fig 3: 0301), A431 skin cancer cells (Fig. 4: 0401), A549 lung adenocarcinoma cells (Fig. 5: 0501), MOLT-4 leukemia cells (Fig. 6), and COLO201 colon cancer cells (Fig. 7). On the other hand, cytotoxic activation was hardly shown under the same conditions in regards to normal cells of the said tissues. When specific explanations are given, for example, in regards to dosing of glycosphingolipid derivatives up to 200 µM, fluctuation of the percentage of living cells can hardly be noted for CS-HC (0302) in Fig. 3. However, in regards to dosing of glycosphingolipid derivatives of more than 100 µM, the percentage of living cells can be seen to have been remarkably delinked for Hep-G2 (0301) in Fig. 3. Moreover, cytotoxic activation was also discovered in TIG (0402) with regard to normal skin cells through dosing of glycosphingolipid derivatives of more than 100 µM in Fig. 4. At a concentration of 150 µM, slightly over 80% of TIG cells were living cells. On the other hand, slightly over 30% of A431 (0401) skin cancer cells were living cells. There was a large difference there between of about 50%. According to the discovery as such, the glycosphingolipid derivatives of the present invention showed strong concentration-dependent antitumor activity in regards to the aforementioned tumor cells within a prescribed scope. At the same time, the glycosphingolipid derivatives hardly showed activity on normal cells. Alternatively, even when activity was shown, there was a large difference between such activity and activity on tumor cells. Therefore, it was revealed to be possible to use at least the glycosphingolipid derivatives of the present invention as antitumor agents with fewer side-effects in regards to the tumor cells mentioned above.

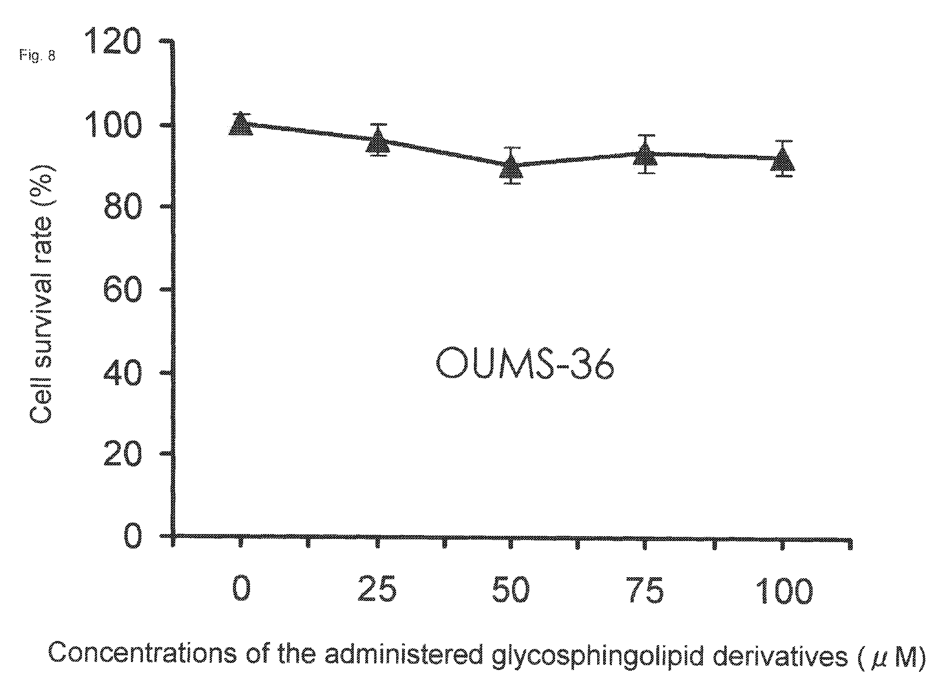

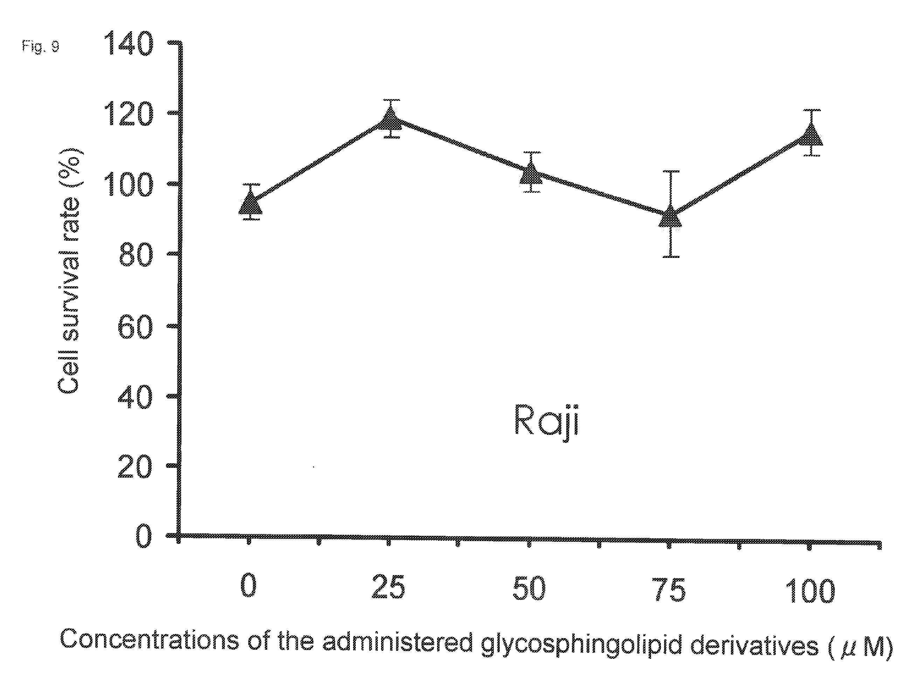

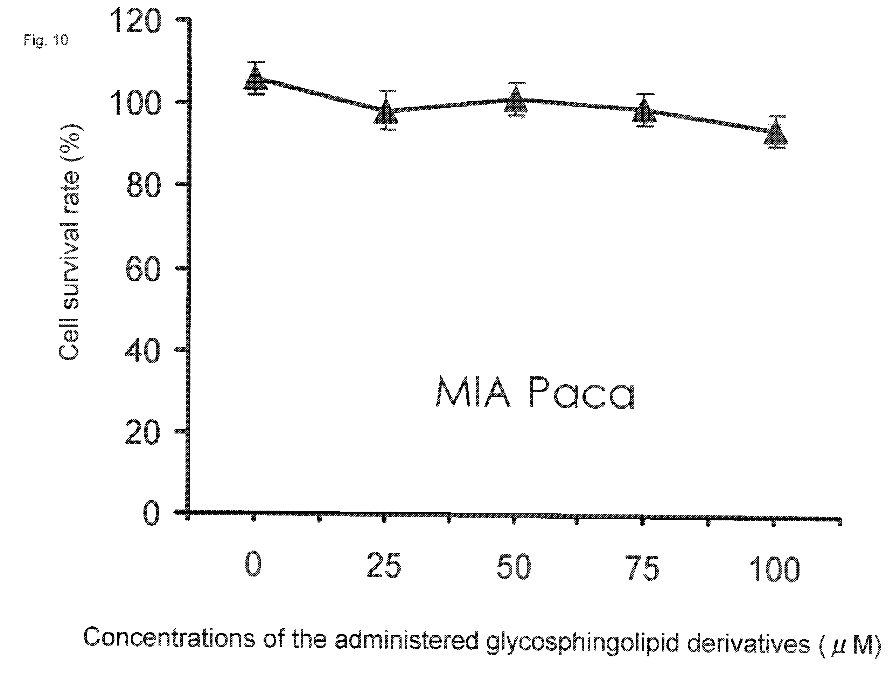

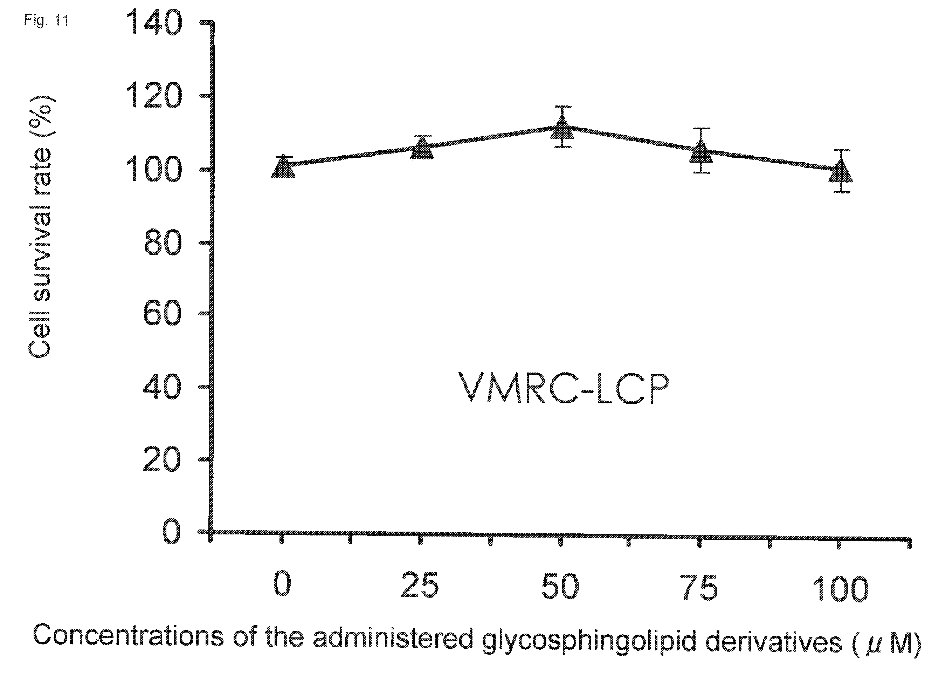

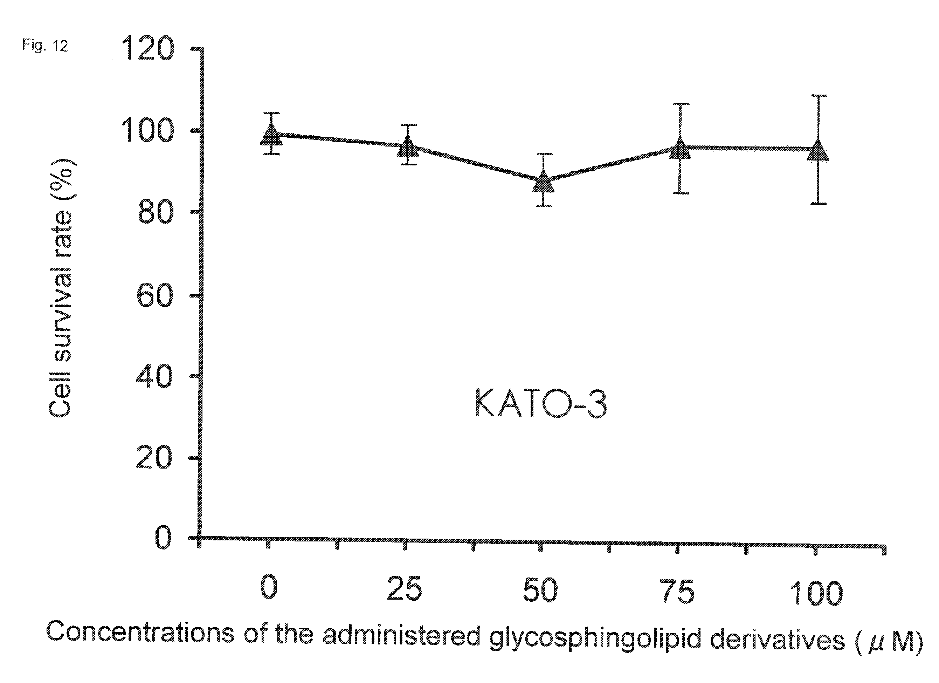

The glycosphingolipid derivatives of the present invention did not show cytotoxic activation against normal fibroblasts (Fig. 8), lymphoma cells (Fig.9), pancreas cancer cells (Fig. 10), squamous cell carcinoma cells of the lung (Fig. 11), or gastric cancer cells (Fig. 12) under the conditions of the embodiment. That it to say, it is possible to think that the glycosphingolipid derivatives of the present invention cannot be expected to be effective as antitumor agents in regards to such tumors. However, this fact does not reduce effects of the antitumor agents at all. The fact that the glycosphingolipid derivatives of the present invention show strong cytotoxic activation only for specific tumor cells means that while the glycosphingolipid derivatives attack the targets accurately, they have fewer side-effects on normal cells and other cells. Thus, it is indicated that the glycosphingolipid derivatives of the present invention are more useful than the conventional glycosphingolipid derivatives, which showed cytotoxic activation against all tumors.

### Third embodiment

<Immunostimulatory activity with administration of the glycosphingolipid derivatives of the present invention>

### (Object)

It was verified whether or not the glycosphingolipid derivatives of the present invention had the function of activating NKT cells within a living organism in the same manner as the case of αGalcer.

### (Method for experiment)

C57BL/6 8^{th}-week mice were used. 20 µg, 100 µg, and 200 µg/mouse of the glycosphingolipid derivatives that were purified in the first embodiment were administered to the abdominal cavities of the aforementioned mice, respectively. Additionally, individuals that received 2 µg/mouse were used as positive controls. After dosing, lymphocytes were separated from the livers of the mice over time. Double labeling immunostaining was undertaken with 2 types of monoclonal antibodies, FITC-labeled anti-CD3 (Pharmingen) and PE-labeled anti-NK1.1 (Pharmingen). Thereafter, flow cytometric analysis (FACSCalibur, Becton-Dickinson) was undertaken for dynamic states of NKT cells. In addition, a method of lymphocyte separation from the livers and the immunostaining method were subject to the methods of Non-Patent Document 7.

### (Results)

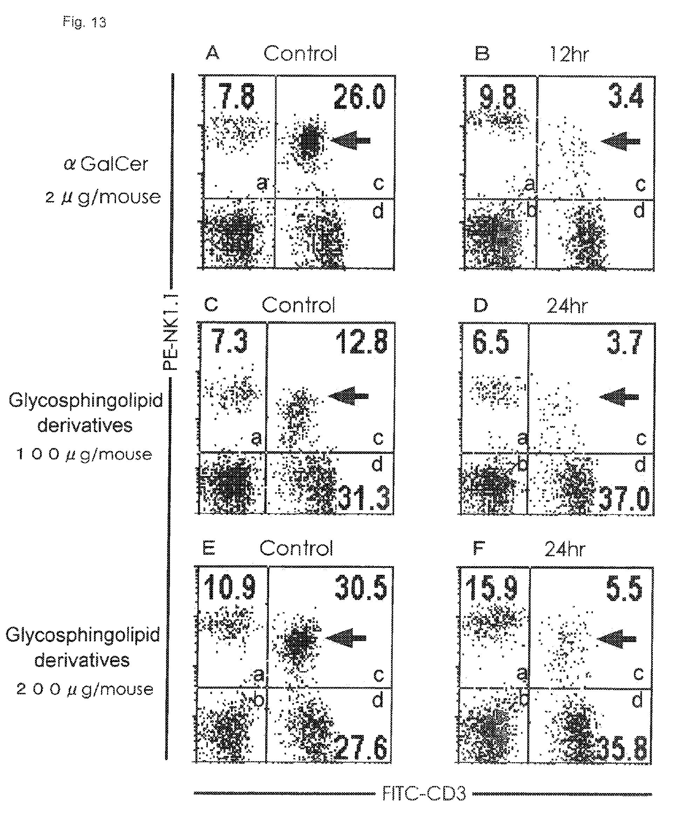

Cytograms from analysis results based on flow cytometry mentioned above are shown in Fig. 13. In this Fig., cytograms of lymphocytes for mice to which αGalcer as the positive control (A and B), 100 µg of the glycosphingolipid derivatives (C and D), and 200 µg of the glycosphingolipid derivatives (E and F) were administered are shown, Moreover, A, C, and E show the results for non-dosed mice, B shows results 12 hours after administration, and D and F show results 24 hours after administration. Cytograms show fluorescence intensities for FITC-labeled anti-CD3 on the horizontal axis and PE-labeled anti-NK1.1 antibody on the vertical axis in a log scale fashion. The larger the fluorescence intensities for FITC, the greater the number of CD3 molecules on the surfaces of cells. And the larger the PE fluorescence intensities, the greater the number of NK1.1 molecules on the measured surfaces of cells. Based on 2 types of fluorescence intensities, 4 fractions of cytograms are divided into "a" as NK cell fractions (CD3⁻NK1.1⁺), "b" (not shown in some cytograms due to difference of dot) as fractions such those of B cells, macrophages, and the like (CD3⁻NK1.1⁻), "c" as fractions of NKT cells (CD3⁺NK1.1⁺)(shown by arrows in the Fig.), and "d" as the fractions of T cells (CD3⁺NK1.1⁻). Additionally, numeric values within the fractions represent the percentages of cells included in such fractions relative to all measured cells.

First, in regards to mice to which αGalCer as the positive control were administered, most NKT cells in the liver disappeared within 12 hours following dosing (B). As not shown in the Fig., however, such tendency had not changed even after 24 hours following administration. NKT cells have 2 functions: cytotoxic activation that directly attacks targeted cells in the same manner as in the case of NK cells, and discharging of cytokine within the cells induced by activation (Non-Patent Document 1). That is to say, disappearance of NKT cells of the liver means that the NKT cells that were activated by αGalcer experienced apoptosis and disappeared.

On the other hand, in regards to administration of 20 µg/mouse of the glycosphingolipid derivatives of the present invention (not shown in the Fig.), the effects seen with αGalcer could not be observed even after 24 hours. Additionally, in regards to administration of 100 µg/mouse and 200 µg/mouse of the glycosphingolipid derivatives of the present invention, remarkable effects could not be observed after 12 hours, either (not shown in Fig.). However, disappearance of NKT cells of the liver as shown in D and F was observed in 24 hours. As such, it was revealed that the glycosphingolipid derivatives of the present invention had the function of activating NKT cells gradually at high concentrations. Additionally, apoptosis accompanying activation of NKT cells is explained in detail with reference to the sixth embodiment.

### Fourth embodiment

<Verification of INF-γ in the blood due to administration of the glycosphingolipid derivatives of the present invention>

### (Object)

It is known that NKT cells produce INF-γ due to αGalcer activation (Non-Patent Documents 4 and 5). That is to say, INF-γ in the blood increases due to administration of αGalcer. Therefore, it was revealed whether or not the level of INF-γ in the blood would be changed due to administration of the glycosphingolipid derivatives of the present invention.

### (Method for experiment)

Mice prepared in the third embodiment were used. After about 1 ml of blood had been collected from each mouse over time, serum was separated. The level of INF-γ in such serum was measured in accordance with the ELISA test. In regards to the ELISA test, a BD Opt EIA set (Pharmingen) was used, and the method was subject to the attached protocol.

### (Results)

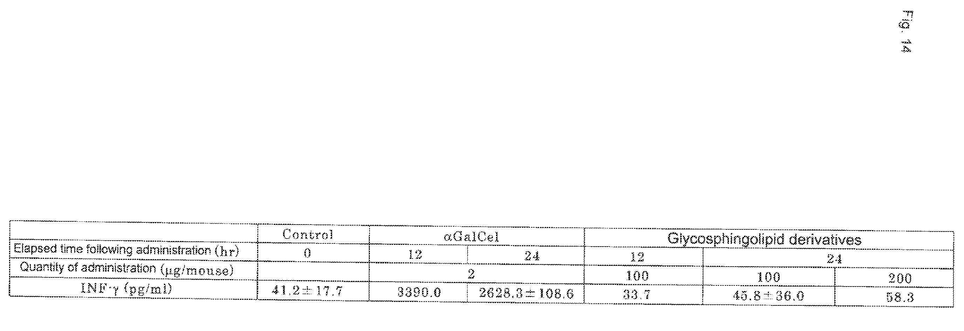

Results are shown in Fig. 14. Due to administration of αGalcer, the level of INF-γ in the blood increased from about 40 pg/ml for a normal mouse to about 3,000 pg/ml. On the other hand, in regards to administration of the glycosphingolipid derivatives of the present invention, almost the same values as those for normal mice were shown. That is to say, it was clarified that despite the fact that the glycosphingolipid derivatives of the present invention activated NKT cells as shown in the third embodiment, the level of INF-γ in the blood did not rise. It can be thought that this is due to differences in action mechanisms regarding activation of NKT cells between the glycosphingolipid derivatives of the present invention and αGalcer.

### Fifth embodiment

<Verification of liver damage due to administration of the glycosphingolipid derivatives of the present invention>

### (Object)

αGalcer shows antitumor activity through administration thereof to a body due to its strong cytotoxic activation. And it is also known that αGalcer is problematic in that it causes cytotoxicity to the liver at the same time (Non-Patent Document 1). As stated in the third embodiment, it is necessary to administer the glycosphingolipid derivatives of the present invention at much higher concentrations than in the case of αGalcer for activation of NKT cells. If so, it is possible to think that there could be a possibility of inducing the same level of (or greater) severe liver damage as in the case of αGalcer. Therefore, verification was made as to induction of liver damage resulting from administration of the glycosphingolipid derivatives of the present invention to a body.

### (Method for experiment)

The mice prepared in the third embodiment were used. After collecting about 1 ml of blood from each mouse over time, serums were separated. Transaminase values in the serums (GPT) were measured using of a Transaminase CII Test Wako (Wako Pure Chemical Industries, Ltd) as an extracorporeal diagnostic kit. The method for measurement was subject to the protocol attached with the kit. In addition, generally speaking, red cells of mice are easily hemolyzed. Thus, GOT values regarding which errors easily occur to results of measurements are not presented herein.

### (Results)

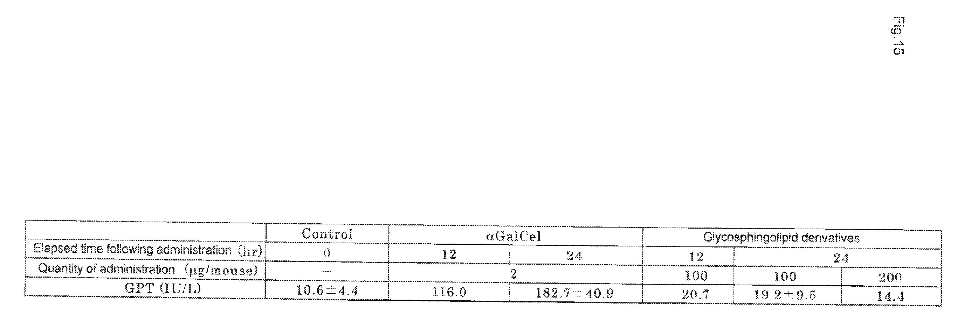

Results of measurement of GPT values are shown in Fig. 15. Herein, first, brief explanations are given in regards to GPT in the serums. GPT normally functions in liver cells. Due to destruction of stem cells caused by liver damage, such as hepatitis and the like, GPT leaks into the blood. Thus, increase of GPT values in the blood is an index of liver damage. Generally, it is understood that liver damage, such as hepatitis and the like, exists when the figures are greater than 100IU (international unit)/L (liter).

As shown in Fig. 15, GPT values for normal mice are about 11IU/L. GPT values due to administration of αGalcer show high values of more than 100 U/L, at 116IU/L after 12 hours and 183 IU/L after 24 hours. It was recognized that liver damage was caused as reported thus far. On the other hand, in regards to GPT values resulting from administration of the glycosphingolipid derivatives of the present invention, even with administration of extremely high concentrations thereof (20 µg), it was shown that GPT values were slightly higher than values of normal mice, which were less than 20 IU/L. Such GPT values were low. This indicates that the glycosphingolipid derivatives of the present invention hardly result in cytotoxic activation against liver cells. That is to say, the glycosphingolipid derivatives of the present invention have much fewer side-effects.

### Sixth embodiment

<Verification of apoptosis induction of NKT cells based on the glycosphingolipid derivatives of the present invention>

### (Object)

It is known that NKT cells cause apoptosis themselves accompanying αGalcer activation (Non-Patent Document 1). Therefore, it was verified whether or not apoptosis of NKT cells was caused even as a result of activation by the glycosphingolipid derivatives of the present invention.

### (Method for experiment)

The basic method for the experiment is the same as that of the third embodiment. C57BL/6 8^{th}-week mice were used. 200 µg/mouse of the glycosphingolipid derivatives that were purified in the first embodiment were administered to the abdominal cavities of the aforementioned mice. And 2µg/mouse of αGalcer was likewise administered. After 24 hours following the dosing, lymphocytes had been separated from the livers of the mice over time. Triple labeling immunostaining was undertaken by fluorescently-labeled monoclonal antibody based on 2 types of FITC-labeled anti-CD3 antibody (Pharmingen) and PE-labeled anti-NK1.1 antibody (Pharmingen) for detection of NKT cells (CD3⁺NK1.1 ⁺), and FITC labeled Annexin V (Pharmingen) for detection of apoptosis cells. The method for immunostaining was subject to that of Non-Patent Document 1. Next, PerCP and PE were detected via flow cytometric analysis (FACSCalibur, Becton-Dickinson). Based on the fluorescence intensity thereof, NKT cells' fractions (areas shown in c of Fig. 13) were obtained. These processes were the same as those of the third embodiment, other than with regard to the types of fluorescent substances. According to the embodiment, cells which caused apoptosis within the fractions were detected via measurement of fluorescence intensity of FITC in such fractions. In addition, the same operation was undertaken for T cell fractions (areas shown in d of Fig. 13) as controls.

### (Results)

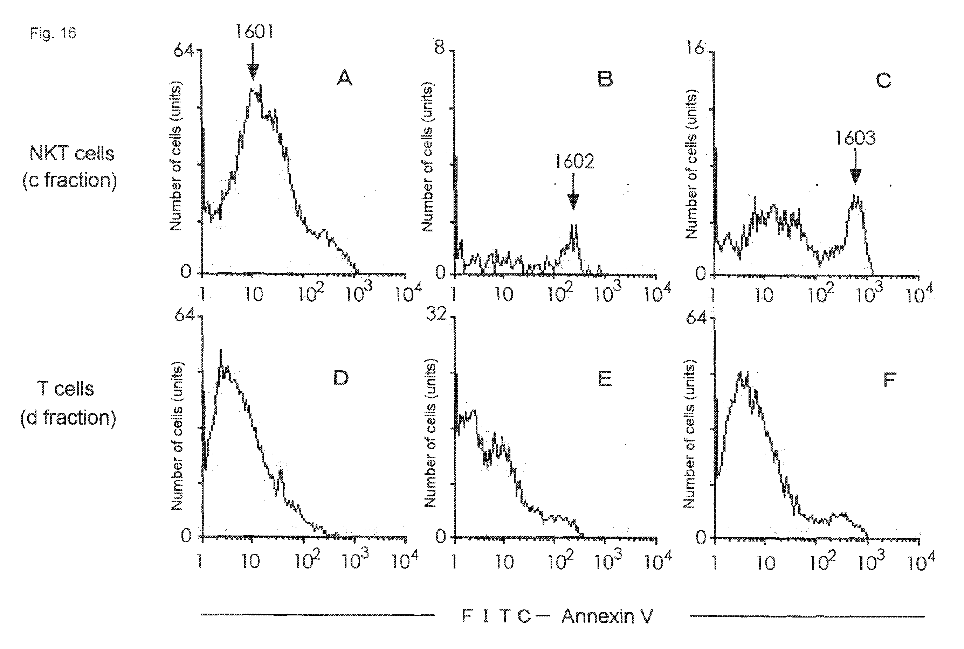

The results of measurement are shown in Fig. 16. Histograms of mouse NKT cell fractions and T cell fractions of unprocessed normal mouse cells are shown (A and D). Histograms of mouse NKT cell fractions and T cell fractions to which 2 µg/mouse of αGalcer was administered are shown (B and E). Histograms of mouse NKT cell fractions and T cell fraction to which 200 µg/mouse of the glycosphingolipid derivatives of the present invention was administered are shown (C and F). Additionally, A, B, and C represent NKT cell fractions, and D, E, and F represent T cell fractions. The fluorescence intensity of fluorescent substance C is shown on the horizontal axis and the number of cells (in units) is shown on the vertical axis for each profile. Annexin V detects cells that have fallen under the initial state of apoptosis. Thus, when FITC fluorescence intensity is strong in cells -- that is to say, when cells are Annexin V positive -- apoptosis is positive. Cells that are Annexin V positive result in a state of necrosis and reach complete death eventually (PI positive).

In regards to normal mice A, NKT cells have a weak peak of fluorescence intensity (1601) of around 10. On the other hand, in regards to mice B to which αGalcer was administered, such peak completely disappeared. And a peak (1602) could be observed for only a short while at a relatively high fluorescence intensity of close to 200, which was comparatively strong. In relation to T cells as a control, almost no difference in patterns of histograms for D and E could be found. Apoptosis induction could not be recognized. Thus, this indicates that NKT cells were activated through administration of αGalcer, and furthermore, that cell membranes were destroyed via escalation of apoptosis. In regards to the glycosphingolipid derivatives of the present invention, the peak of weak fluorescence intensity shown in A was dissolved. In lieu thereof, the peak (1603) occurred at the location of strong fluorescence intensity around 600. In regards to D and F for T cells as a control, hardly any differences in histogram patterns could be observed, and apoptosis induction could not be recognized. Therefore, in the same manner as in the case of αGalcer, it was revealed that NKT cells were activated via administration of glycosphingolipid derivatives, and apoptosis of NKT cells was induced. However, in regards to apoptosis of NKT cells via the glycosphingolipid derivatives, the peak remained in a location of strong fluorescence intensity. Therefore, compared with αGalcer apoptosis induction, it is possible to think that apoptosis induction of NKT cells via glycosphingolipid derivatives progressed in a comparatively gradual manner.

### Seventh embodiment

<Verification of antitumor activity against cancer cells via the glycosphingolipid derivatives of the present invention and of liver damage accompanying the same>

### (Object)

It was verified whether or not the glycosphingolipid derivatives of the present invention have functions of suppressing proliferation of cancer cells within a living body.

### (Method for experiment)

C3H/HeJJcl 4^{th}-week mice were used. About 1 x 10⁴ units of mouse liver cancer cells (MH134 -TC) were implanted in such mice intracutaneously. 2 weeks following such implantation, mice were divided into 2 groups. 0.2 mg of the glycosphingolipid derivatives purified through the first embodiment was administered to the abdominal cavities of only one group thereof each day. Cancer cell rate was obtained as the rate of increase of intracutaneous areas of cancer cells. GPT values in the serums were measured as an index of side-effects. In addition, the same measurement was conducted for one group to which the glycosphingolipid derivatives were not administered at all as a control.

### (Results)

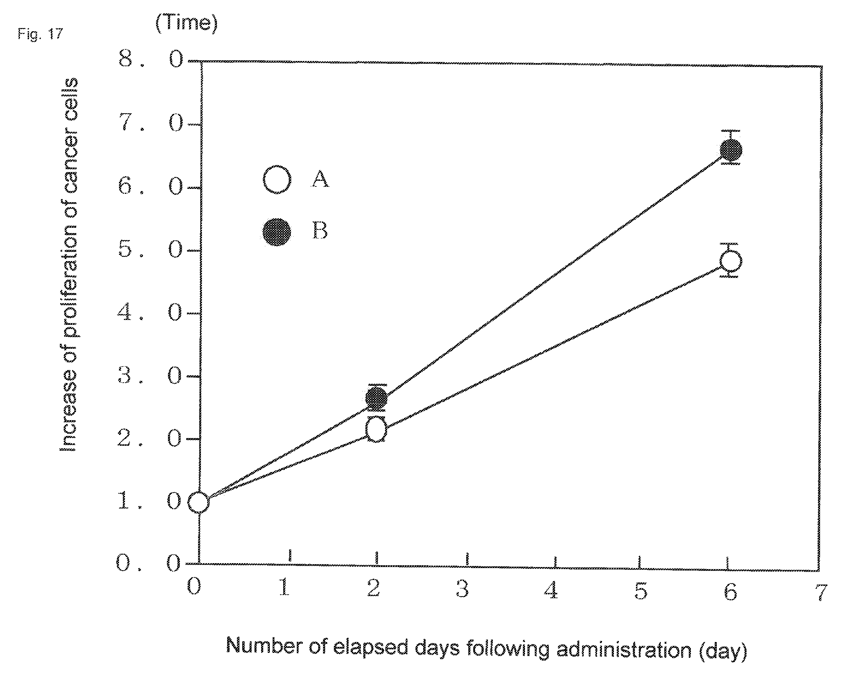

Results regarding suppressive effects against proliferation of cancer cells via administration of the glycosphingolipid derivatives are shown in Fig. 17. The elapsed number of days following administration of the glycosphingolipid derivatives is shown on the horizontal axis and relative values in regards to areas of cancer tissues at the time of commencement of administration of the glycosphingolipid derivatives are shown on the vertical axis. A shows the results for mice of the group to which the glycosphingolipid derivatives were administered. B shows the results for mice of the control group. Additionally, GPT value measurement results are shown in Table 2.

**[Table 2]**

| | GPT |
|---|---|
| Non-dosed mice | 25.1 |
| Dosed mice | 24.8+3.2 |

Minor differences can be observed in Fig. 17 in regards to the rate of increase of intracutaneous areas of cancer cells from the 2^{nd} day following administration of the glycosphingolipid derivatives. On the 6^{th} day, figures for one group (B) as a control increased about 6.8 times. On the other hand, figures for one group (A) to which the glycosphingolipid derivatives were administered increased about 4.9 times (p < 0.01). Due to such fact, it was indicated that the glycosphingolipid derivatives significantly suppressed proliferation of cancer cells within a living body.

Additionally, in Table 2, no large difference was discovered concerning GPT values in regards to one group to which the glycosphingolipid derivatives were administered and one group as a control. This indicates that the glycosphingolipid derivatives of the present invention hardly have cytotoxic activation against liver cells. That is so say, they have extremely fewer side-effects.

Putting it all together, it is indicated that the glycosphingolipid derivatives of the present invention hardly exert actions against normal cells, and selectively exhibit cytotoxic activation against cancer cells as the tumor cells.

### Eighth embodiment

<Verification of metastasis suppression of cancer cells within a living body via the glycosphingolipid derivatives of the present invention and individual survival rate>

### (Object)

Metastasis suppression of cancer cells within a living body via administration of the glycosphingolipid derivatives of the present invention and fluctuation of individual survival rate were verified.

### (Method for experiment)

C57BL/6 8^{th}-week mice were used. 0.2 mg/mouse of the glycosphingolipid derivatives which were purified in the first embodiment was administered to abdominal cavities of the aforementioned mice. 200 µg/mouse thereof was administered again three days later. At the same time, about 1 x 10⁵ units of mouse lymphoma cells EL4 was transferred from tail veins, and the rate of survival was measured. Additionally, in regards to the mice processed under the same conditions as those mentioned above, after about 5 x 10⁶ units of EL4 mouse lymphoma cells were injected thereinto, livers were extracted on the 21^{st} day following the injection. The numbers of EL4 cell mass units transferred to such livers were gauged visually. In addition, the same measurement was undertaken for mice to which the glycosphingolipid derivatives were not administered as a control.

### (Results)

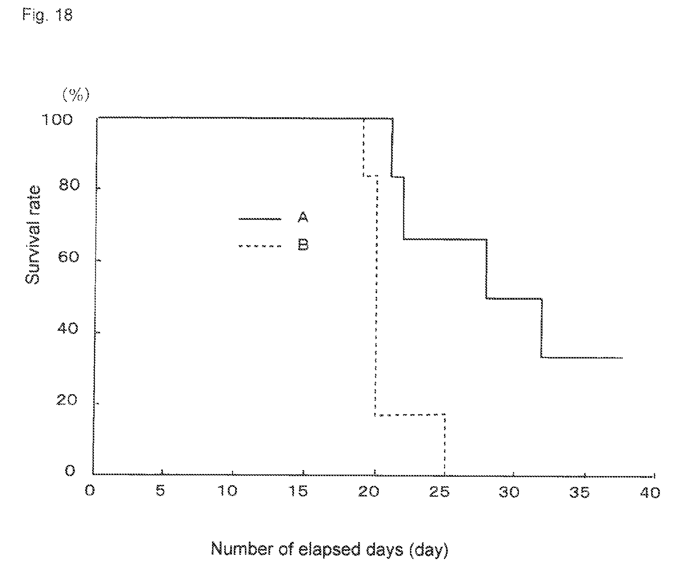

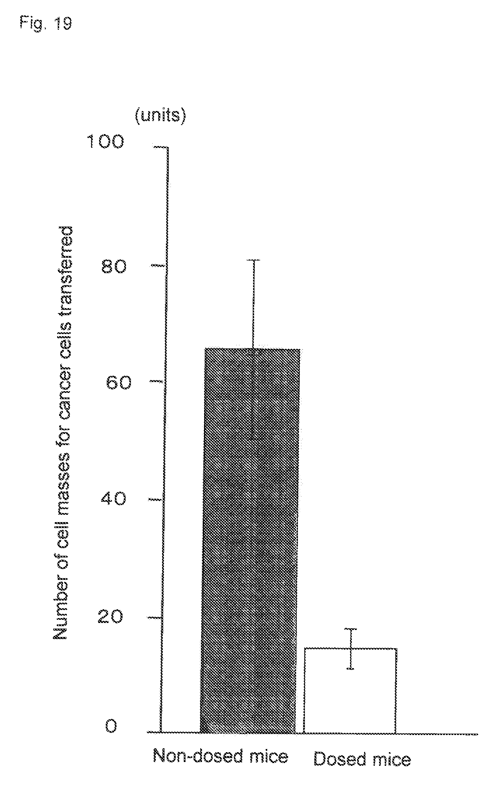

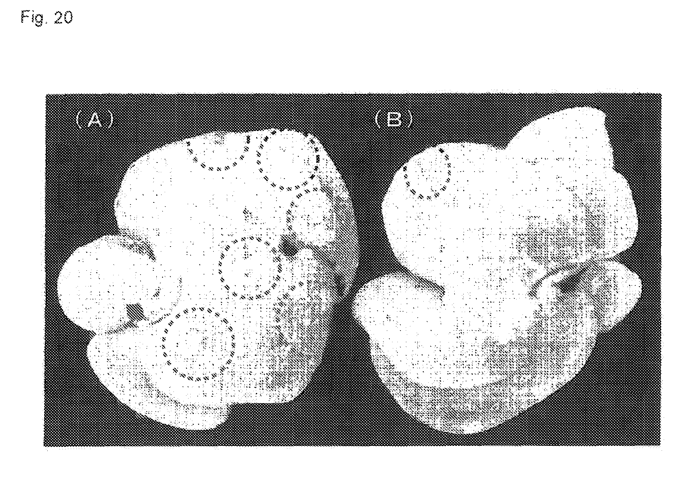

The survival rates for mice to which the glycosphingolipid derivatives were administered and mice to which the glycosphingolipid derivatives were not administered are shown in Fig. 18. The elapsed number of days following injection of EL4 cells is shown on the horizontal axis and the survival rate (%) of mice (n=6) is shown on the vertical axis. Fig. 19 shows the results for the number of mass cells transferred to livers of mice to which the glycosphingolipid derivatives were administered and mice to which the glycosphingolipid derivatives were not administered on the 21^{st} day following the injection of EL4. The number of mass cells (units) transferred regarding EL4 cells is shown on the vertical axis. Additionally, Fig. 20 indicates the livers extracted from mice to which the glycosphingolipid derivatives were not administered (A) and mice to which the glycosphingolipid derivatives were administered (B) on the 21^{st} day following the injection of EL4. Macular traces within the dashed circle indicate EL4 cell mass that was transferred.

In Fig. 18, in regards to mice to which the glycosphingolipid derivatives were administered (A), after the elapse of 20 days following the day of transferring of EL4 cells, the rate of survival started to decline. However, about 30% (p < 0.05) survived even after the elapse of 35 days. On the other hand, in regards to mice as controls (B), on the 20^{th} day following transferring of EL4, the rate of survival remarkably declined. All mice were dead on the 25^{th} day. Due to such results, it was indicated that the glycosphingolipid derivatives significantly increased the rate of survival for individuals with infiltrating cancer cells.

In Fig. 19, the number of cell masses regarding EL4 cells that were transferred to livers of mice to which the glycosphingolipid derivatives were administered was about 15. On the other hand, a number of cell masses of about 65 resulted for the mice used as controls (p < 0.05). Moreover, according to Fig. 20, transferring of EL4 cells to livers could hardly be observed in regards to mice to which the glycosphingolipid derivatives were administered (B). Due thereto, it was shown that the glycosphingolipid derivatives significantly suppressed the transferring of such cells.

A such, it can be thought that the glycosphingolipid derivatives of the present invention activate NKT cells as immunostimulating agents within a living body, suppress transferring of tumor cells, and increase the rate of survival.

### [Brief Description of Drawings]

Fig. 1 is a conceptual diagram that explains the process for extraction from beer.
Fig. 2 is an example of a flow chart of the method for extracting of glycosphingolipid derivatives and the like from natural raw materials.
Fig. 3 shows survival curves for liver cells (Hep G2, CS-HC) to which the glycosphingolipid derivatives of the present invention were administered.
Fig. 4 shows survival curves for skin cells (A431, TIG) to which the glycosphingolipid derivatives of the present invention were administered.
Fig. 5 shows survival curves for lung cells (A549, W38, W138 Sub) to which the glycosphingolipid derivatives of the present invention were administered.
Fig. 6 shows survival curves for Molt-4 leukemia cells to which the glycosphingolipid derivatives of the present invention were administered.
Fig.7 shows survival curves for COLO201 colon cancer cells to which the glycosphingolipid derivatives of the present invention were administered.
Fig. 8 shows survival curves for OUMS36 normal fibroblasts to which the glycosphingolipid derivatives of the present invention were administered.
Fig. 9 shows survival curves for Raji lymphoma cells to which the glycosphingolipid derivatives of the present invention were administered.
Fig. 10 shows survival curves for MIA Paca pancreas cancer cells to which the glycosphingolipid derivatives of the present invention were administered.
Fig. 11 shows survival curves for VMRC-LCP squamous cell carcinoma cells of the lung to which the glycosphingolipid derivatives of the present invention were administered.
Fig. 12 shows survival curves for KATO-3 gastric cancer cells to which the glycosphingolipid derivatives of the present invention were administered.
Fig. 13 shows cytograms that indicate dynamic states of liver NKT cells over time resulting from administration of the glycosphingolipid derivatives of the present invention or aGalCer.
Fig. 14 shows the INF-γ level in the blood of mice resulting from administration of the glycosphingolipid derivatives of the present invention or αGalCer.
Fig. 15 shows GPT values in the blood of mice resulting from administration of the glycosphingolipid derivatives of the present invention or αGalCer.
Fig. 16 shows hitrograms that show apoptosis induction in regards to liver NKT cells and
T cells resulting from administration of the glycosphingolipid derivatives of the present invention or αGalCer.
Fig. 17 shows rising curves for areas of cancer cells of the mice to which the glycosphingolipid derivatives of the present invention were administered.
Fig. 18 shows rates of survival relating to mouse lymphoma for the mice to which the glycosphingolipid derivatives of the present invention were administered and the mice to which the glycosphingolipid derivatives of the present invention were not administered.
Fig. 19 shows graphs of the number of cell masses for cancer cells transferred concerning the mice to which the glycosphingolipid derivatives of the present invention were administered and the mice to which the glycosphingolipid derivatives of the present invention were not administered.
Fig. 20 is a diagram showing transfer of cancer cells to livers for the mice to which the glycosphingolipid derivatives of the present invention were administered and the mice to which the glycosphingolipid derivatives of the present invention were not administered.
Explanations of codes

0301: Hep G2 survival curve
0301: CS-HC survival curve

## Claims

1. A medical composition, which comprises more than one glycosphingolipid derivative represented by the following formula (1) as an active ingredient. 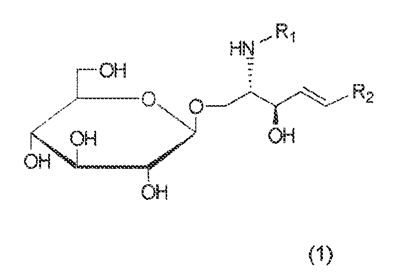 wherein R₁ represents a residue in which monocarbonic acid is combined with a carboxyl group in an acid amide combination, and R₂ is (CH₂)_{X1} CH=CH(CH₂)_{X2} CH₃ (where X₁ and X₂ corresponds to 0 or to a natural number and X₁ + X₂ = 10).

2. A medical composition, which comprises more than one glyrosphingolipid derivative represented by the following formula (2) as an active ingredient. 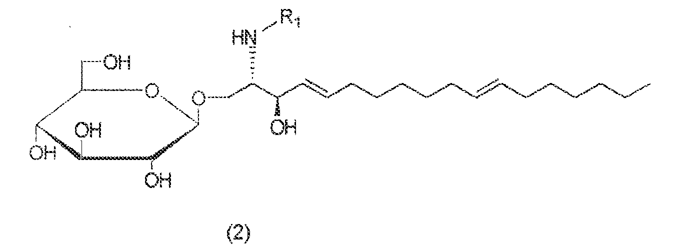 wherein R₁ represents a residue in which monocarbonic acid is combined with a carboxyl group in an acid amide combination.

3. A medical composition, which comprises more than one glycosphingolipid derivative represented by the following formula (3) as an active ingredient 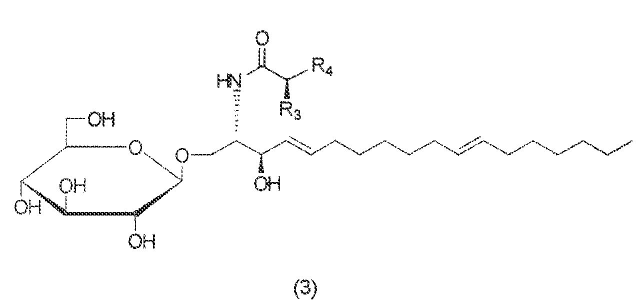 wherein R₃ represents H or OH and R₄ corresponds to either the following (a) or (b):
(a) when R₃ is H, R₄ is (CH₂)₁₃ CH₃ or (CH₂)₆CH=CHCH₂CH=CH(CH₂)₄ CH₃; and
(b) when R₃ is OH, R₄ is (CH₂)_{Y} CH₃ (where Y is an integer from 13 - 21) or (CH₂)_{Z1} CH=CH(CH₂)_{X2} CH₃. (where Z₁ and Z₂ corresponds to 0 or to a natural number and Z₁ + Z₂ =19).

4. The medical composition according to claim 3, wherein (b) is defined as follows:
(b) When R₃ is OH, R₄ is (CH₂)_{Y} CH₃ (where Y is an integer from 13 - 21) or (CH₂)₁₂ CH=CH(CH₂)₇CH₃.

5. The medical composition according to any one of claims 1 to 4, wherein the glycosphingolipid derivatives are natural glycosphingolipid derivatives.

6. The medical composition according to any one of claims 1 to 4, wherein the glycosphingolipid derivatives are extracted from brewers' grains obtained through a process for manufacturing of beers and the like.

7. The medical composition according to any one of claims 1 to 6, wherein the medical composition is used for an antitumor agent.

8. The medical composition according to claim 7, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above formula (1) as an active ingredient:

9. The medical composition according to claim 7, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above formula (2) as an active ingredient:

10. The medical composition according to claim 7, which exhibits selective cytotoxic activation against tumor cells corresponding to any of colon cancer cells, liver cancer cells, skin cancer cells, lung adenocarcinoma cells, or leukemia cells and which comprises more than one glycosphingolipid derivative represented by the above formula (3) as an active ingredient.

11. The medical composition according to any one of claims 1 to 6, such medical composition being used for an immunostimulating agent.
